(19) Europäisches Patentamt
European Patent Office
Office européen des brevets

(11) Numéro de publication: **0 392 922 A2**

(12) **DEMANDE DE BREVET EUROPEEN**

(21) Numéro de dépôt: **90400988.3**

(51) Int. Cl.[5]: **A61K 31/415, A61K 31/42**

(22) Date de dépôt: **11.04.90**

(30) Priorité: **12.04.89 FR 8904792**

(43) Date de publication de la demande:
**17.10.90 Bulletin 90/42**

(84) Etats contractants désignés:
**AT BE CH DE DK ES FR GB GR IT LI LU NL SE**

(71) Demandeur: **Laboratoires DELAGRANGE**
**1, Avenue Pierre-Brossolette**
**F-91380 Chilly-Mazarin(FR)**

(72) Inventeur: **Acher, Jacques**
**32, route de Saint Vrain**
**F-91760 Itteville(FR)**
Inventeur: **Monier, Jean-Claude**
**6, rue de la Sorbonne**
**F-91510 Lardy(FR)**
Inventeur: **Schmitt, Jean-Paul**
**13, rue Victor Hugo**
**F-91290 Arpajon(FR)**
Inventeur: **Gardaix-Luthereau**
**9, rue Guichard**
**F-94230 Cachan(FR)**
Inventeur: **Costall, Brenda**
**The Old Rectory, Addingham**
**Ilkey, West Yorkshire(GB)**
Inventeur: **Naylor, Robert**
**The Old Rectory, Addingham**
**Ilkey, West Yorkshire(GB)**

(54) **Application de dérivés de 2-méthoxy 4-[4,5-dihydro 2-imidazolyl (ou 2-oxazolyl)amino]5-chloro benzamide dans le traitement des troubles de la fonction cognitive.**

(57) L'invention concerne l'utilisation de dérivés de benzamide de formule :

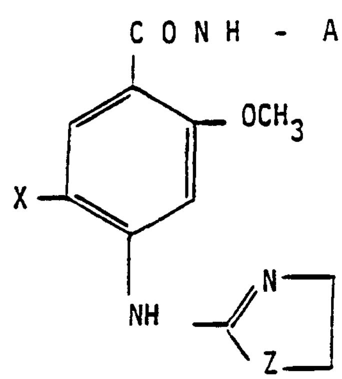

(I)

dans laquelle :
- A représente un groupe diéthylaminoéthyle ou un groupe de formule :

EP 0 392 922 A2

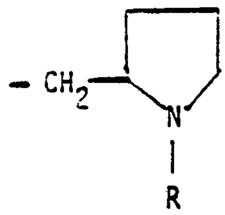

où R est un groupe $C_1$-$C_3$ alkyle, allyle, cyclopropylméthyle ou cyclohexénylméthyle,
- X représente un atome de chlore ou de brome,
- Z représente un groupe NH ou un atome d'oxygène, avec la condition suivante :
lorsque Z est un atome d'oxygène, A est un groupe diéthylaminoéthyle,
et de leurs sels pharmacologiquement acceptables pour la préparation de médicaments utiles dans le traitement et la prévention des troubles de la fonction cognitive.

**Application de dérivés de 2-méthoxy 4- [ 4,5-dihydro 2-imidazolyl (ou 2-oxazolyl ) amino] 5-halogéno benzamide dans le traitement des troubles de la fonction cognitive.**

La présente invention concerne une nouvelle application de dérivés de benzamide, de formule (I)

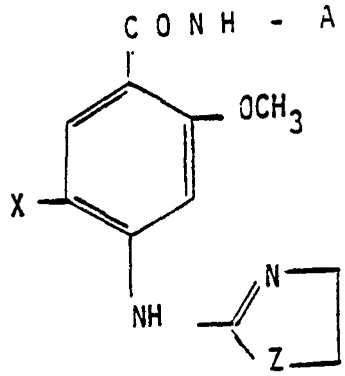

(I)

dans laquelle :
- A représente un groupe diéthylaminoéthyle ou un groupe de formule :

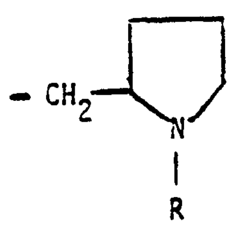

où R est un groupe $C_1$-$C_3$-alkyle, allyle, cyclopropylméthyle ou cyclohexénylméthyle,
- X représente un atome de chlore ou de brome,
- Z représente un groupe NH ou un atome d'oxygène,
avec la condition suivante :
lorsque Z est un atome d'oxygène, A est un groupe diéthylaminoéthyle, et de leurs sels pharmacologiquement acceptables.

L'invention concerne en particulier les composés suivants :

composé 1 : N-[2(diéthylamino)éthyl]2-méthoxy 4-[(1H-4,5-dihydro 2-imidazolyl) amino]5-chloro benzamide.

Composé 2 : N-(1-allyl 2-pyrrolidinylméthyl)2-méthoxy 4-[(1H-4,5-dihydro 2-imidazolyl) amino]5-chloro benzamide.

Composé 3 : N-(1-méthyl 2-pyrrolidinylméthyl) 2-méthoxy 4-[(1H-4,5-dihydro 2-imidazolyl) amino]5-chloro benzamide.

Composé 4 : N-[2-(diéthylamino) éthyl]2-méthoxy 4-[(4,5-dihydro 2-oxazolyl) amino]5-chloro benzamide.

Composé 5 : N-(1-cyclopropylméthyl 2-pyrrolidinylméthyl) 2-méthoxy 4-[(1H-4,5-dihydro 2-imidazolyl) amino]5-chloro benzamide.

Composé 6 : N-(1-cyclohexénylméthyl 2-pyrrolidinyl méthyl) 2-méthoxy 4-[(1H-4,5-dihydro 2-imidazolyl) amino]5-chloro benzamide.

Composé 7 : N-[2-(diéthylamino) éthyl] 2-méthoxy 4-[(1H-4,5-dihydro 2-imidazolyl) amino]5-bromo benzamide.

Composé 8 : N-(1-propyl 2-pyrrolidinylméthyl) 2-méthoxy 4-[(1H-4,5-dihydro 2-imidazolyl) amino]5-chloro benzamide.

Les composés de la présente invention sont connus d'après la demande de brevet français publiée sous le n° 2592042, où ils sont décrits ainsi qu'une série de composés de formule voisine, comme

activants du système nerveux central.

Certains de ces composés ont également révélé des propriétés gastromotrices qui ont fait l'objet de la demande de brevet français n° 88 16433 ou des propriétés anxiolytiques et antipsychotiques qui ont fait l'objet de la demande de brevet européen publiée sous le n° 0295350 et de la demande de brevet français n° 88 16764.

Poursuivant ses recherches, la demanderesse a découvert que les composés de formule (I) telle que définie ci-dessus, étaient doués d'une propriété spécifique et totalement inattendue : l'amélioration des fonctions cognitive et mnésique.

Cette particularité a été mise en évidence par des tests pharmacologiques pratiqués sur le rongeur et le primate notamment par un test d'habituation chez la souris et par un test d'apprentissage chez le singe marmouset.

I - TEST D'HABITUATION CHEZ LA SOURIS ADULTE JEUNE

Dans une boîte divisée, par une cloison percée, en 2 compartiments, l'un blanc brillamment éclairé, l'autre noir et faiblement éclairé, on place des souris naturellement habituées à un environnement obscur au milieu du compartiment blanc. Pour fuir cette agression, elles se réfugient dans le compartiment sombre. Au début, elles explorent le compartiment blanc, et il leur faut 8 à 15 secondes pour repérer l'ouverture entre les 2 compartiments. On répète l'expérience pendant plusieurs jours et elles "apprennent" à fuir, sans exploration préalable du compartiment blanc. Après 5 à 6 jours, la fuite est immédiate et elles explorent plus longuement le compartiment sombre.

L'habituation se caractérise donc par une diminution du temps nécessaire au passage du compartiment blanc au compartiment sombre et par une augmentation de l'exploration au compartiment sombre (augmentation du nombre de redressements et de déplacements) au détriment du compartiment blanc.

Ce processus d'habituation est altéré par administration de scopolamine.

CONDUITE DU TEST

Sur des souris mâles albinos, pesant 25-30g, on mesure sous surveillance video, chaque jour, pendant 5 minutes :

1) le temps de latence avant le passage du compartiment blanc vers le compartiment sombre.

2) le nombre de redressements exploratoires dans chacun des compartiments, durant le test.

3) le nombre de lignes franchies sur le plancher quadrillé des 2 compartiments, traduisant la locomotion exploratoire.

4) le pourcentage de temps passé dans le compartiment sombre.

Ces mesures ont été faites sur des groupes de souris recevant par voie sous-cutanée, soit un véhicule inerte soit un composé selon l'invention, administré deux fois par jour à dose subanxiolytique (1ng/kg pour le composé 1, 10ng/kg pour les composés 2 à 4, 1µg/kg pour les composés 5 et 6 et 10µg/kg pour le composé 7).

L'effet de l'administration aiguë de scopolamine, injectée par voie intrapéritonéale à la dose de 0,25mg/kg, a été étudié chez le groupe témoin et chez les groupes traités, après acquisition de l'habituation.

L'effet du composé 1 de l'invention (1ng/kg par voie sous-cutanée, 2 fois par jour) sur l'altération de l'habituation provoquée par administration chronique de scopolamine (0,25mg/kg par jour) a également été étudié.

Les mêmes mesures ont été réalisées avec des composés de formule proche de celle des composés de la présente invention.

Ces composés décrits également dans la demande de brevet français publiée sous le n° 2592092, ont été utilisés dans le cas présent, comme composés de référence.

Il s'agit des composés suivants :

- composé de référence A : N-(1-éthyl 2-pyrrolidinylméthyl) 2-méthoxy 3,5-dibromo 4-[(1H-4,5-dihydro 2-imidazolyl) amino] benzamide.
- composé de référence B : N-[2-(diéthylamino) éthyl]2-méthoxy 4-[(1H-4,5-dihydro 4-méthyl 2-imidazolyl) amino]5-chloro benzamide.

- composé de référence C : N-[2-(diéthylamino) éthyl]2-méthoxy 3,5-dichloro 4-[(1H-4,5-dihydro 2-imidazolyl) amino]benzamide.

Ces trois composés ont été administrés deux fois par jour, par voie sous- cutanée, à la dose de 10ng/kg. Les mêmes essais ont été réalisés avec les composés A et B, à la dose de 1µg/kg.

RESULTATS DU TEST

Les résultats de l'étude de l'habituation et de l'effet de l'administration aigue de scopolamine sont indiqués dans les tableaux 1 à 13 et représentés sur les figures 1 à 13.

Dans le groupe témoin, on a trouvé que le temps de latence moyen de 11 secondes au début, décroît progressivement jusqu'à un minimum moyen de 3,4 secondes atteint au bout de 5 jours.

Parallèlement le temps passé dans le compartiment noir augmente progressivement pour atteindre environ 78% au bout de 5 jours, le nombre de déplacements et de redressements dans ce compartiment augmentant également tandis que le temps de séjour et le nombre de déplacements et de redressements dans le compartiment blanc diminuent. L'ensemble de ces résultats révèle le processus d'habituation qui se stabilise en 5 jours.

L'administration de scopolamine à la dose de 0,25mg/kg inhibe ce phénomène d'habituation. L'inhibition est mise en évidence par un temps de latence accru, devenant même supérieur au temps de latence initial, par une diminution du temps de séjour dans le compartiment noir ainsi que par une diminution du nombre de déplacements et de redressements dans ce compartiment.

Dans les groupes de souris traitées par un composé de l'invention, on a constaté que le temps de latence diminuait plus vite, que sa valeur minimum était en général plus faible et qu'elle était obtenue plus rapidement que dans le groupe témoin.

On a constaté également que le temps de séjour dans le compartiment noir ainsi que le nombre de déplacements et de redressements dans ce compartiment augmentaient de façon significative dès le deuxième ou le troisième jour, par rapport au premier jour et par rapport au groupe témoin.

Enfin; l'administration de scopolamine n'a pas ou a peu modifié l'ensemble des paramètres étudiés.

Dans les groupes traités par les composés de référence, le processus d'habituation s'est révélé analogue à celui du groupe témoin et l'administration de scopolamine a provoqué les mêmes effets.

Il apparaît donc que les composés de l'invention améliorent le processus d'habituation et préviennent les perturbations induites par la scopolamine, contrairement aux composés de référence de structure très proche.

Les résultats de l'étude des effets du composé 1 sur l'altération de l'habituation due à une administration chronique de scopolamine, sont représentés sur la figure 14.

Cette étude a montré que l'administration du composé 1 de l'invention, après inhibition de l'habituation par administration quotidienne de scopolamine, permettait de rétablir le comportement acquis avant les perturbations induites par la scopolamine.

Les composés selon l'invention améliorent donc la fonction cognitive chez la souris et préviennent ou inhibent les détériorations de cette fonction, causées par exemple par une perturbation de l'activité cholinergique centrale.

TABLEAU 1

**HABITUATION DE SOURIS ADULTES JEUNES EFFET DE L'ADMINISTRATION AIGUE DE SCOPOLAMINE**

| TRAITEMENT | JOUR | COMPARTIMENT BLANC | | COMPARTIMENT NOIR | | % TEMPS DANS NOIR | LATENCE BLANC→ NOIR (sec) |
|---|---|---|---|---|---|---|---|
| | | redressements en 5 minutes | déplacements en 5 minutes | redressements en 5 minutes | déplacements en 5 minutes | | |
| Véhicule | 1 | 30 | 37 | 66 | 68 | 55 | 11 |
| Véhicule | 2 | 29 | 39 | 65 | 69 | 61 | 10,4 |
| Véhicule | 3 | 28 | 35 | 67 | 64 | 66 | 11,1 |
| Véhicule | 4 | 24 | 33 | 71 | 70 | 72 | 9,3 |
| Véhicule | 5 | 13 | 19 | 83 | 87 | 78 | 3,4 |
| Véhicule + scopolamine | 6 | 74+ | 87+ | 19+ | 20+ | 22+ | 20,2+ |

Véhicule : voie S.C., 2 fois par jour - Scopolamine : 0,25mg/kg/I.P.

n = 5 - E.S.M. < 10,4% - + P < 0,001 (altération de l'apprentissage par la scopolamine).

TABLEAU 2

**HABITUATION DE SOURIS ADULTES JEUNES EFFET DE L'ADMINISTRATION AIGUE DE SCOPOLAMINE**

| TRAITEMENT | JOUR | COMPARTIMENT BLANC | | COMPARTIMENT NOIR | | % TEMPS DANS NOIR | LATENCE BLANC→ NOIR (sec) |
|---|---|---|---|---|---|---|---|
| | | redressements en 5 minutes | déplacements en 5 minutes | redressements en 5 minutes | déplacements en 5 minutes | | |
| Composé 1 | 1 | 31 | 34 | 67 | 67 | 56 | 10 |
| Composé 1 | 2 | 22 | 31 | 56 | 68 | 74* | 7,4 |
| Composé 1 | 3 | 9* | 11* | 90* | 90* | 76 | 2,6* |
| Composé 1 | 4 | 10* | 9* | 89* | 92* | 78 | imméd.* |
| Composé 1 | 5 | 5 | 16 | 89 | 90 | 75 | 0,5 |
| Composé 1 + scopolamine | 6 | 11° | 12° | 86° | 90° | 75° | 0,7° |

Composé 1 : 1ng/kg/S.C., 2 fois par jour - Scopolamine : 0,25 mg/kg/I.P.

n = 5 - E.S.M. < 13,5% - * P < 0,001 (apprentissage significatif par rapport au groupe témoin).

° P < 0,001 (antagonisme de l'altération de l'apprentissage par la scopolamine).

TABLEAU 3

**HABITUATION DE SOURIS ADULTES JEUNES EFFET DE L'ADMINISTRATION AIGUE DE SCOPOLAMINE**

| TRAITEMENT | JOUR | COMPARTIMENT BLANC | | COMPARTIMENT NOIR | | % TEMPS DANS NOIR | LATENCE BLANC→ NOIR (sec) |
|---|---|---|---|---|---|---|---|
| | | redressements en 5 minutes | déplacements en 5 minutes | redressements en 5 minutes | déplacements en 5 minutes | | |
| Composé 2 | 1 | 26 | 41 | 58 | 70 | 57 | 12 |
| Composé 2 | 2 | 18 | 25* | 73* | 85* | 67* | 4* |
| Composé 2 | 3 | 8* | 11* | 99* | 97* | 73* | 3* |
| Composé 2 | 4 | 9* | 10* | 98* | 115* | 72* | 2* |
| Composé 2 | 5 | 10* | 16* | 87* | 89* | 69* | 2* |
| Composé 2 + scopolamine | 6 | 10* ° | 13* ° | 98* ° | 102* ° | 67* ° | 2* ° |

Composé 2 : 10ng/kg/S.C., 2 fois par jour - Scopolamine : 0,25mg/kg/IP.

n = 5 - E.S.M. < 11,7% - * P < 0,001 (apprentissage significatif par rapport à J 1).

° P < 0,001 (antagonisme de l'altération de l'apprentissage par la scopolamine).

TABLEAU 4

**HABITUATION DE SOURIS ADULTES JEUNES EFFET DE L'ADMINISTRATION AIGUE DE SCOPOLAMINE**

| TRAITEMENT | JOUR | COMPARTIMENT BLANC | | COMPARTIMENT NOIR | | % TEMPS DANS NOIR | LATENCE BLANC→ NOIR (sec) |
|---|---|---|---|---|---|---|---|
| | | redressements en 5 minutes | déplacements en 5 minutes | redressements en 5 minutes | déplacements en 5 minutes | | |
| Composé 3 | 1 | 24 | 31 | 53 | 67 | 52 | 10 |
| Composé 3 | 2 | 21 | 26 | 57 | 63 | 54 | 10 |
| Composé 3 | 3 | 13* | 19* | 79* | 80* | 65* | 3* |
| Composé 3 | 4 | 13* | 10* | 73* | 76* | 64* | 5* |
| Composé 3 | 5 | 14* | 21* | 72* | 77* | 65* | 4* |
| Composé 3 + scopolamine | 6 | 12*° | 14*° | 89*° | 93*° | 70*° | 3*° |

Composé 3 : 10ng/kg/S.C., 2 fois par jour - Scopolamine : 0,25mg/kg/IP.

n = 5 - E.S.M. < 12,7% - * P < 0,05 - P < 0,001 (apprentissage significatif par rapport à J 1).

° P < 0,001 (antagonisme de l'altération de l'apprentissage par la scopolamine).

TABLEAU 5

**HABITUATION DE SOURIS ADULTES JEUNES EFFET DE L'ADMINISTRATION AIGUE DE SCOPOLAMINE**

| TRAITEMENT | JOUR | COMPARTIMENT BLANC | | COMPARTIMENT NOIR | | % TEMPS DANS NOIR | LATENCE BLANC→ NOIR (sec) |
|---|---|---|---|---|---|---|---|
| | | redressements en 5 minutes | déplacements en 5 minutes | redressements en 5 minutes | déplacements en 5 minutes | | |
| Composé 4 | 1 | 28 | 36 | 71 | 80 | 51 | 12 |
| Composé 4 | 2 | 14* | 20* | 110* | 111* | 77* | 2* |
| Composé 4 | 3 | 10* | 15* | 101* | 104* | 67* | 2* |
| Composé 4 | 4 | 8* | 7* | 138* | 139* | 69* | 1* |
| Composé 4 | 5 | 9* | 8* | 103* | 117* | 73* | immédiat* |
| Composé 4 + scopolamine | 6 | 10*° | 13*° | 121*° | 123*° | 79*° | 2*° |

Composé 4 : 10ng/kg/S.C., 2 fois par jour - Scopolamine : 0,25mg/kg/I.P.

n = 5 E.S.M. < 11,7% - * P < 0,001 (apprentissage significatif par rapport à J 1).

° P < 0,001 (antagonisme de l'altération de l'apprentissage par la scopolamine).

TABLEAU 6

**HABITUATION DE SOURIS ADULTES JEUNES EFFET DE L'ADMINISTRATION AIGUE DE SCOPOLAMINE**

| TRAITEMENT | JOUR | COMPARTIMENT BLANC | | COMPARTIMENT NOIR | | % TEMPS DANS NOIR | LATENCE BLANC→ NOIR (sec) |
|---|---|---|---|---|---|---|---|
| | | redressements en 5 minutes | déplacements en 5 minutes | redressements en 5 minutes | déplacements en 5 minutes | | |
| Composé 5 | 1 | 23 | 33 | 69 | 71 | 59 | 11 |
| Composé 5 | 2 | 9* | 11* | 83* | 92* | 76* | 2* |
| Composé 5 | 3 | 6* | 11* | 89* | 96* | 82* | 3* |
| Composé 5 | 4 | 7* | 9* | 90* | 94* | 80* | 2* |
| Composé 5 | 5 | 5* | 8* | 92* | 103* | 79* | 2* |
| Composé 5 + scopolamine | 6 | 7° | 11° | 87° | 91° | 81° | 6° |

Composé 5 : 1 µg/kg/S.C., 2 fois par jour - Scopolamine : 0,25mg/kg/I.P.

n=5 - E.S.M. < 19% - * P < 0,05 - P < 0,001 (apprentissage significatif par rapport à $J_1$)

° P < 0,001 (antagonisme de l'altération de l'apprentissage par la scopolamine)

TABLEAU 7

| HABITUATION DE SOURIS ADULTES JEUNES EFFET DE L'ADMINISTRATION AIGUE DE SCOPOLAMINE | | | | | | | |
|---|---|---|---|---|---|---|---|
| TRAITEMENT | JOUR | COMPARTIMENT BLANC | | COMPARTIMENT NOIR | | % TEMPS DANS NOIR | LATENCE BLANC→ NOIR (sec) |
| | | redressements en 5 minutes | déplacements en 5 minutes | redressements en 5 minutes | déplacements en 5 minutes | | |
| Composé 6 | 1 | 22 | 32 | 70 | 73 | 56 | 10 |
| Composé 6 | 2 | 11* | 9* | 86* | 101* | 80* | 3* |
| Composé 6 | 3 | 9* | 11* | 90* | 96* | 76* | 2* |
| Composé 6 | 4 | 6* | 9* | 81* | 103* | 78* | 2* |
| Composé 6 | 5 | 6* | 9* | 86* | 94* | 79* | 3* |
| Composé 6 + scopolamine | 6 | 7° | 9° | 92° | 100° | 82° | 4° |

Composé 6 : 1 µg/kg/S.C., 2 fois par jour - Scopolamine : 0,25mg/kg/I.P.

n=5 - E.S.M. < 21% - * P < 0,005 - P < 0,001 (apprentissage significatif par rapport à $J_1$)

° P < 0,001 (antagonisme de l'altération de l'apprentissage par la scopolamine)

TABLEAU 8

| HABITUATION DE SOURIS ADULTES JEUNES EFFET DE L'ADMINISTRATION AIGUE DE SCOPOLAMINE | | | | | | | |
|---|---|---|---|---|---|---|---|
| TRAITEMENT | JOUR | COMPARTIMENT BLANC | | COMPARTIMENT NOIR | | % TEMPS DANS NOIR | LATENCE BLANC→ NOIR (sec) |
| | | redressements en 5 minutes | déplacements en 5 minutes | redressements en 5 minutes | déplacements en 5 minutes | | |
| Composé 7 | 1 | 24 | 37 | 70 | 82 | 56 | 10 |
| Composé 7 | 2 | 11* | 12* | 96* | 111* | 76* | 2* |
| Composé 7 | 3 | 6* | 12* | 111* | 120* | 83* | 0* |
| Composé 7 | 4 | 9* | 8* | 121* | 106* | 74* | 2* |
| Composé 7 | 5 | 9* | 12* | 103* | 101* | 72* | 1,5* |
| Composé 7 + scopolamine | 6 | 5° | 11° | 112° | 120° | 70° | 3° |

Composé 7 : 10 µg/kg/S.C., 2 fois par jour - Scopolamine : 0,25mg/kg/I.P.

n=5 - E.S.M. < 20% - * P < 0,005 - P < 0,001 (apprentissage significatif par rapport à $J_1$)

° P < 0,001 (antagonisme de l'altération de l'apprentissage par la scopolamine).

TABLEAU 9

| HABITUATION DE SOURIS ADULTES JEUNES EFFET DE L'ADMINISTRATION AIGUE DE SCOPOLAMINE | | | | | | | |
|---|---|---|---|---|---|---|---|
| TRAITEMENT | JOUR | COMPARTIMENT BLANC | | COMPARTIMENT NOIR | | % TEMPS DANS NOIR | LATENCE BLANC→ NOIR (sec) |
| | | redressements en 5 minutes | déplacements en 5 minutes | redressements en 5 minutes | déplacements en 5 minutes | | |
| Ref. A | 1 | 28 | 27 | 61 | 71 | 53 | 10 |
| Ref. A | 2 | 25 | 28 | 63 | 62 | 52 | 11 |
| Ref. A | 3 | 24 | 31 | 54 | 73 | 54 | 10 |
| Ref. A | 4 | 23 | 26 | 63 | 70 | 56 | 6 |
| Ref. A | 5 | 14* | 14* | 74* | 86* | 67* | 3* |
| Ref. A + scopolamine | 6 | 82+ | 88+ | 14+ | 19+ | 27+ | 17+ |

Composé de référence A = 10ng/kg/S.C., deux fois par jour - Scopolamine : 0,25mg/kg/I.P.

n = 5. E.S.M. < 12,6% - * P < 0,05 - P < 0,001 (apprentissage significatif par rapport à J 1)

+ P < 0,01 - P < 0,001 (altération de l'apprentissage par la scopolamine)

TABLEAU 10

**HABITUATION DE SOURIS ADULTES JEUNES EFFET DE L'ADMINISTRATION AIGUE DE SCOPOLAMINE**

| TRAITEMENT | JOUR | COMPARTIMENT BLANC | | COMPARTIMENT NOIR | | % TEMPS DANS NOIR | LATENCE BLANC→ NOIR (sec) |
|---|---|---|---|---|---|---|---|
| | | redressements en 5 minutes | déplacements en 5 minutes | redressements en 5 minutes | déplacements en 5 minutes | | |
| Ref. A | 1 | 22 | 30 | 69 | 79 | 59 | 12 |
| Ref. A | 2 | 27 | 40 | 62 | 76 | 59 | 11 |
| Ref. A | 3 | 29 | 36 | 70 | 80 | 59 | 10 |
| Ref. A | 4 | 20 | 32 | 73 | 84 | 60 | 5 |
| Ref. A | 5 | 10* | 16* | 92* | 96* | 77* | 3* |
| Ref. A + scopolamine | 6 | 76+ | 80+ | 19+ | 23+ | 19+ | 22,5+ |

Composé de référence A = 1 µg/kg/S.C., deux fois par jour - Scopolamine : 0,25mg/kg/I.P.

n=5 - E.S.M. < 26% - * P < 0,05 - P < 0,001 (apprentissage significatif par rapport à $J_1$)

+ P < 0,001 (altération de l'apprentissage par la scopolamine).

TABLEAU 11

| HABITUATION DE SOURIS ADULTES JEUNES EFFET DE L'ADMINISTRATION AIGUE DE SCOPOLAMINE | | | | | | | |
|---|---|---|---|---|---|---|---|
| TRAITEMENT | JOUR | COMPARTIMENT BLANC | | COMPARTIMENT NOIR | | % TEMPS DANS NOIR | LATENCE BLANC→ NOIR (sec) |
| | | redressements en 5 minutes | déplacements en 5 minutes | redressements en 5 minutes | déplacements en 5 minutes | | |
| Ref. B | 1 | 23 | 24 | 68 | 90 | 56 | 11 |
| Ref. B | 2 | 26 | 32 | 53 | 81 | 54 | 9 |
| Ref. B | 3 | 25 | 26 | 61 | 83 | 52 | 10 |
| Ref. B | 4 | 22 | 25 | 63 | 80 | 51 | 8 |
| Ref. B | 5 | 11* | 16* | 81* | 97 | 67* | 3* |
| Ref. B + scopolamine | 6 | 19+ | 92+ | 18+ | 26+ | 21+ | 16+ |

Composé de référence B : 10ng/kg/S.C., 2 fois par jour - Scopolamine : 0,25mg/kg/I.P.

n = 5 . E.S.M. < 12,1% - * P < 0,05 - P < 0,001 (apprentissage significatif par rapport à J 1)

+ P < 0,01 - P < 0,001 (altération de l'apprentissage par la scopolamine).

TABLEAU 12

**HABITUATION DE SOURIS ADULTES JEUNES EFFET DE L'ADMINISTRATION AIGUE DE SCOPOLAMINE**

| TRAITEMENT | JOUR | COMPARTIMENT BLANC | | COMPARTIMENT NOIR | | % TEMPS DANS NOIR | LATENCE BLANC→ NOIR (sec) |
|---|---|---|---|---|---|---|---|
| | | redressements en 5 minutes | déplacements en 5 minutes | redressements en 5 minutes | déplacements en 5 minutes | | |
| Ref. B | 1 | 27 | 36 | 71 | 83 | 57 | 12 |
| Ref. B | 2 | 29 | 42 | 76 | 80 | 63 | 13,5 |
| Ref. B | 3 | 26 | 33 | 80 | 80 | 62 | 11 |
| Ref. B | 4 | 13* | 12* | 86 | 86 | 72* | 7 |
| Ref. B | 5 | 11* | 12* | 94* | 97* | 79* | 3* |
| Ref. B + scopolamine | 6 | 63 + | 57 + | 30 + | 20 + | 20 + | 17,5 + |

Composé de référence B : 1 µg/kg/S.C., 2 fois par jour - Scopolamine : 0,25mg/kg/I.P.

n=5 - E.S.M. < 18,5% - * P < 0,05 - P < 0,001 (apprentissage significatif par rapport à $J_1$)

\+ P < 0,001 (altération de l'apprentissage par la scopolamine).

TABLEAU 13

| HABITUATION DE SOURIS ADULTES JEUNES EFFET DE L'ADMINISTRATION AIGUE DE SCOPOLAMINE | | | | | | | |
|---|---|---|---|---|---|---|---|
| TRAITEMENT | JOUR | COMPARTIMENT BLANC | | COMPARTIMENT NOIR | | % TEMPS DANS NOIR | LATENCE BLANC→ NOIR (sec) |
| | | redressements en 5 minutes | déplacements en 5 minutes | redressements en 5 minutes | déplacements en 5 minutes | | |
| Ref. C | 1 | 29 | 43 | 56 | 63 | 52 | 11 |
| Ref. C | 2 | 30 | 35 | 57 | 67 | 56 | 10 |
| Ref. C | 3 | 25 | 43 | 51 | 61 | 52 | 7 |
| Ref. C | 4 | 28 | 37 | 51 | 63 | 53 | 9 |
| Ref. C | 5 | 18* | 29* | 72* | 81* | 67* | 3* |
| Ref. C + scopolamine | 6 | 90 + | 86 + | 21 + | 26 + | 23 + | 20 + |

Composé de référence C : 10ng/kg/S.C., 2 fois par jour - Scopolamine : 0,25mg/kg/I.P.

n = 5 - E.S.M. < 12,9% - * P < 0,05 - P < 0,001 (apprentissage significatif par rapport à $J_1$)

+ P < 0,001 (altération de l'apprentissage par la scopolamine)

II- TEST D'HABITUATION CHEZ LA SOURIS AGEE

Ce test a été réalisé avec un appareil identique et dans des conditions analogues au test d'habituation chez la souris jeune tel que décrit en I.

Les mêmes paramètres (temps de latence, nombre de déplacements et de redressements dans chacun des compartiments et pourcentage de temps passé dans le compartiment sombre) ont été mesurés et les effets d'une injection de scopolamine ont été déterminés. Les résultats ont été comparés à ceux qui ont été obtenus avec un groupe de souris jeunes.

Les souris âgées étant particulièrement sensibles aux effets de la scopolamine, la scopolamine a été injectée à la dose de 0,1 mg/kg au lieu de 0,25 mg/kg pour le groupe des souris jeunes.

Enfin, l'effet du composé 1 de l'invention (administré 2 fois par jour à la dose de 10ng/kg) sur l'habituation et sur les perturbations induites par la scopolamine chez la souris âgée a été déterminé par comparaison avec les résultats obtenus avec un groupe témoin de souris âgées.

RESULTATS DU TEST

Les résultats sont indiqués dans les tableaux 14 à 16. D'après les résultats figurant dans le tableau 14, il apparaît que les souris âgées, contrairement aux souris jeunes, évoluent peu dans leur faculté d'apprécier la disponibilité d'un compartiment sombre. Bien qu'il y ait une certaine habituation, les résultats indiquent que, même en situation de tests prolongée, les souris âgées manifestent des troubles de la mémoire associative.

Dans la mesure où les possibilités d'apprentissage des souris âgées sont très inférieures à celles des souris jeunes, les perturbations causées par la scopolamine sont relativement moins importantes (tableau 15). Cependant ces perturbations se produisent à une dose faible qui n'aurait aucun effet sur les souris jeunes.

D'après les résultats du tableau 16, il apparaît que le traitement par le composé 1 de l'invention améliore considérablement l'apprentissage des souris âgées, leurs performances devenant équivalentes à celles des souris jeunes, et prévient les perturbations de l'habituation causées par la scopolamine.

TABLEAU 14

PROCESSUS D'HABITUATION DE LA SOURIS AGEE COMPARAISON AVEC LA SOURIS ADULTE JEUNE

| | JOUR | COMPARTIMENT BLANC | | COMPARTIMENT NOIR | | % TEMPS DANS NOIR | LATENCE BLANC→ NOIR (sec) |
|---|---|---|---|---|---|---|---|
| | | redressements en 5 minutes | déplacements en 5 minutes | redressements en 5 minutes | déplacements en 5 minutes | | |
| SOURIS AGEES | 1 | 38 | 48 | 43 | 48 | 41 | 12,5 |
| | 2 | 35 | 43 | 46 | 40 | 42 | 10,5 |
| | 3 | 40 | 47 | 40 | 39 | 52 | 10,5 |
| | 4 | 42 | 53 | 38 | 31 | 49 | 10 |
| | 5 | 36 | 39 | 47 | 57 | 56 | 8 |
| | 6 | 24 | 32 | 56 | 61 | 60 | 6 |
| | 7 | 22 | 32 | 55 | 58 | 61 | 6 |
| SOURIS JEUNES | 1 | 23 | 28 | 69 | 80 | 55 | 10 |
| | 2 | 22 | 24 | 72 | 83 | 57 | 11 |
| | 3 | 18 | 23 | 73 | 86 | 65 | 10 |
| | 4 | 9 | 12 | 90 | 96 | 72 | 6 |
| | 5 | 6 | 11 | 93 | 95 | 71 | 4 |
| | 6 | 7 | 12 | 87 | 92 | 75 | 3 |
| | 7 | 5 | 13 | 96 | 94 | 78 | 4 |

E.S.M. < 12,9%

EFFET DE LA SCOPOLAMINE SUR LE PROCESSUS D'HABITUATION DE LA SOURIS AGEE

COMPARAISON AVEC LA SOURIS ADULTE JEUNE

| | TRAITEMENT | JOUR | COMPARTIMENT BLANC | | COMPARTIMENT NOIR | | % TEMPS DANS NOIR | LATENCE BLANC → NOIR (sec) |
|---|---|---|---|---|---|---|---|---|
| | | | redressements en 5 minutes | déplacements en 5 minutes | redressements en 5 minutes | déplacements en 5 minutes | | |
| SOURIS AGEES | | 1 | 40 | 44 | 45 | 52 | 39 | 10 |
| | | 2 | 33 | 49 | 40 | 47 | 40 | 9 |
| | | 3 | 37 | 45 | 38 | 46 | 43 | 9 |
| | | 4 | 32 | 53 | 41 | 48 | 50 | 9 |
| | | 5 | 30 | 39 | 48 | 58 | 52 | 7 |
| | scopolamine (0,1 mg/kg) → | 6 | 51+ | 67+ | 22+ | 27+ | 33+ | 18+ |
| | | 7 | 31 | 40 | 43 | 55 | 51 | 8 |
| SOURIS JEUNES | | 1 | 20 | 25 | 62 | 75 | 51 | 10 |
| | | 2 | 22 | 25 | 69 | 75 | 53 | 11 |
| | | 3 | 20 | 23 | 69 | 82 | 58 | 9 |
| | | 4 | 10 | 12 | 86 | 92 | 67 | 5 |
| | | 5 | 6 | 9 | 91 | 94 | 73 | 4 |
| | scopolamine (0,25mg/kg) → | 6 | 76+ | 78 + | 20 + | 12 + | 27 + | 26 + |
| | | 7 | 7 | 6 | 83 | 82 | 75 | 3 |

n = 5. E.S.M. < 12,6% - +P < 0,05 - P < 0,001 (perturbations par la scopolamine).

TABLEAU 15

## HABITUATION DES SOURIS AGEES

## EFFET DE L'ADMINISTRATION DE SCOPOLAMINE

| | TRAITEMENT | JOUR | COMPARTIMENT BLANC | | COMPARTIMENT NOIR | | % TEMPS DANS NOIR | LATENCE BLANC → NOIR (sec) |
|---|---|---|---|---|---|---|---|---|
| | | | redressements en 5 minutes | déplacements en 5 minutes | redressements en 5 minutes | déplacements en 5 minutes | | |
| SOURIS AGEES GROUPE TEMOIN | | 1 | 39 | 42 | 43 | 50 | 40 | 10 |
| | | 2 | 37 | 45 | 43 | 44 | 42 | 9 |
| | | 3 | 37 | 45 | 40 | 43 | 46 | 9 |
| | | 4 | 36 | 52 | 40 | 44 | 49 | 8 |
| | | 5 | 32 | 35 | 50 | 37 | 52 | 7 |
| | scopolamine (0,1 mg/kg) | 6 | 57 + | 60 + | 18 + | 31 + | 37 + | 12 + |
| | | 7 | 32 | 40 | 46 | 42 | 53 | 8 |
| SOURIS AGEES GROUPE TRAITE | composé 1 | 1 | 30 | 29 | 53 | 78 | 53 | 8 |
| | composé 1 | 2 | 18* | 20* | 84* | 90* | 59* | 3* |
| | composé 1 | 3 | 18* | 21* | 80* | 86* | 62* | 2* |
| | composé 1 | 4 | 18* | 19* | 84* | 82* | 86* | 2* |
| | composé 1 | 5 | 18* | 20* | 81* | 80* | 87* | 1* |
| | composé 1 + scopolamine (0,1 mg/kg) | 6 | 9 ° | 10 ° | 84 ° | 83 ° | 89 ° | 1 ° |
| | composé 1 | 7 | 10* | 11* | 84* | 83* | 84* | 1* |

n = 5 - E.S.M. < 12,8% - * P < 0,001 (apprentissage amélioré) - composé 1 : 10ng/kg, 2 fois par jour .

+ P < 0,05 - P < 0,001 ( perturbations par la scopolamine) - ° P < 0,001 (antagonisme des effets de la scopolamine).

TABLEAU 16

III - TEST D'EVALUATION DE LA FONCTION COGNITIVE CHEZ LE MARMOUSET

Des singes marmousets ont été soumis à une épreuve d'acquisition d'un pouvoir de sélection et à une épreuve d'apprentissage inverse.

Ces épreuves sont réalisées à l'aide d'un appareil connu sous le nom de "Wisconsin General Test Apparatus", mis au point par Harlow en 1949 et décrit dans Psychological Review 56, 51-65. Cet appareil est constitué essentiellement d'une enceinte cubique comportant un tableau pourvu de puits dans lesquels peut être introduit un appât alimentaire. Ces puits peuvent être recouverts par divers objets (stimuli).

Le singe est séparé de l'appareil par un volet opaque qui est ouvert par l'expérimentateur au début de chaque essai. A ce moment là, l'animal peut toucher un stimulus et recevoir la récompense correspondante.

CONDUITE DU TEST

4 singes marmousets mâles et femelles, agês de 15 à 18 mois, ont été utilisés dans cette expérience. L'un des stimuli (stimulus positif) recouvre un puits contenant une récompense alors que l'autre (stimulus négatif) recouvre un puit vide.

Dans un premier temps, les animaux sont entraînés à repérer le stimulus positif qui est toujours le même mais change de position sur le tableau.

Les animaux sont testés pendant des périodes de 4 à 5 jours consécutifs, séparées par des périodes de repos de 2 jours, jusqu'à ce qu'ils obtiennent un résultat de 90 réponses correctes sur 100.

Après une semaine d'interruption, les essais sont poursuivis jusqu'à un résultat de 18 bonnes réponses sur 20 puis 9 bonnes réponses sur 10.

Le protocole observé par la suite est de 5 jours de tests, 2 jours de repos, 5 jours de tests, etc...

Le composé à étudier est administré 2 fois par jour à partir du premier jour de la période de repos. Il y a donc 5 jours de tests sans traitement, 2 jours de traitement sans test puis 5 jours de tests avec traitement.

Les tests ont été réalisés avec le composé 1 de la présente invention, administré 2 fois par jour, par voie sous-cutanée, à la dose subanxiolytique de 10 ng/kg.

Le test est poursuivi jusqu'à ce que 6 essais consécutifs corrects soient enregistrés, le résultat étant représenté par le nombre d'essais réalisés avant l'obtention de ces 6 réponses correctes.

Dans le test d'apprentissage inverse, le stimulus positif du test précédent devient le stimulus négatif et réciproquement. Le résultat est également représenté par le nombre d'essais réalisés avant l'obtention de 6 réponses correctes consécutives.

RESULTATS DU TEST

Les résultats obtenus avec chacun des singes sont représentés sur les figures 15 et 16 (par :

●——●

pour l'acquisition du pouvoir de sélection, par :

○——○

pour l'apprentissage inverse).

Dans la première épreuve, les résultats obtenus avec chacun des singes (singes n°s 1 à 4), de 7-11, 4-12, 3-11 et 8-13 avant traitement passent respectivement à 2-6, 3-9, 0-8 et 3-8 après traitement par le composé 1 de l'invention.

Dans la deuxième épreuve, les résultats de 11-14, 8-15, 6-14 et 9-13 avant traitement, passent

respectivement à 7-12, 8-12, 4-7 et 4-8 après traitement par le composé 1 de l'invention.

Il apparaît que le composé 1 de l'invention améliore l'apprentissage qu'il s'agisse d'une épreuve simple comme celle de l'acquisition de la capacité de sélection ou d'une épreuve difficile comme l'apprentissage inverse.

Ces résultats prouvent l'activité du composé 1 de l'invention dans l'amélioration de la fonction cognitive.

Les résultats obtenus dans l'ensemble des tests pharmacologiques décrits ci-dessus, montrent que les composés selon l'invention peuvent améliorer la fonction cognitive aussi bien que prévenir sa détérioration ou y remédier. Ils peuvent en particulier compenser les troubles de la fonction cognitive dus au vieillissement ce qui laisse envisager leur efficacité dans le traitement de la maladie d'Alzheimer.

## Revendications

1) Utilisation de dérivés de benzamide, de formule (I)

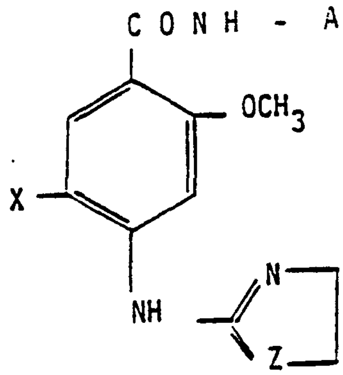

(I)

dans laquelle :
- A représente un groupe diéthylaminoéthyle ou un groupe de formule :

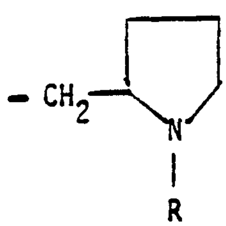

où R est un groupe $C_1$-$C_3$- alkyle, allyle, cyclopropylméthyle ou cyclohexénylméthyle,
- X représente un atome de chlore ou de brome,
- Z représente un groupe NH ou un atome d'oxygène,
avec la condition suivante :
lorsque Z est un atome d'oxygène, A est un groupe diéthylaminoéthyle, et de leurs sels pharmacologiquement acceptables, pour la préparation de médicaments utiles dans le traitment et la prévention des troubles de la fonction cognitive.

2) Utilisation, selon la revendication 1, du N-[2-(diéthylamino) éthyl] 2- méthoxy 4-[(1H-4,5-dihydro 2-imidazolyl) amino]5-chloro benzamide pour la préparation d'un médicament utile pour le traitement et la prévention des troubles de la fonction cognitive.

3) Utilisation selon la revendication 1, du N-(1-allyl 2-pyrrolidinylméthyl) 2-méthoxy 4-[(1H-4,5-dihydro 2-imidazolyl) amino]5-chloro benzamide pour la préparation d'un médicament utile dans le traitement et la prévention des troubles de la fonction cognitive.

4) Utilisation selon la revendication 1, du N-(1-méthyl 2-pyrrolidinylméthyl) 2-méthoxy 4-[(1H-4,5-dihydro 2-imidazolyl) amino]5-chloro benzamide pour la préparation d'un médicament utile dans le traitement et la prévention des troubles de la fonction cognitive.

5) Utilisation selon la revendication 1, du N-[2-(diéthylamino) éthyl] 2-méthoxy 4-[(4,5-dihydro 2-oxazolyl) amino]5-chloro benzamide pour la préparation d'un médicament utile dans le traitement et la prévention des troubles de la fonction cognitive.

6) Utilisation selon la revendication 1, du N-(1-cyclopropylméthyl 2-pyrrolidinylméthyl) 2-méthoxy 4-[-(1H-4,5-dihydro 2-imidazolyl) amino] 5-chloro benzamide pour la préparation d'un médicament utile dans le traitement et la prévention des troubles de la fonction cognitive.

7) Utilisation selon la revendication 1, du N-(1-cyclohexénylméthyl 2-pyrrolidinylméthyl) 2-méthoxy 4-[-(1H-4,5-dihydro 2-imidazolyl) amino] 5-chloro benzamide pour la préparation d'un médicament utile dans le traitement et la prévention des troubles de la fonction cognitive.

8) Utilisation selon la revendication 1, du N-[2-(diéthylamino)éthyl] 2-méthoxy 4-[(1H-4,5-dihydro 2-imidazolyl) amino]5-bromo benzamide pour la préparation d'un médicament utile dans le traitement et la prévention des troubles de la fonction cognitive.

9) Utilisation selon la revendication 1, du N-(1-propyl 2-pyrrolidinyl méthyl) 2-méthoxy 4-[(1H-4,5-dihydro 2-imidazolyl) amino]5-chloro benzamide pour la préparation d'un médicament utile dans le traitement et la prévention des troubles de la fonction cognitive.

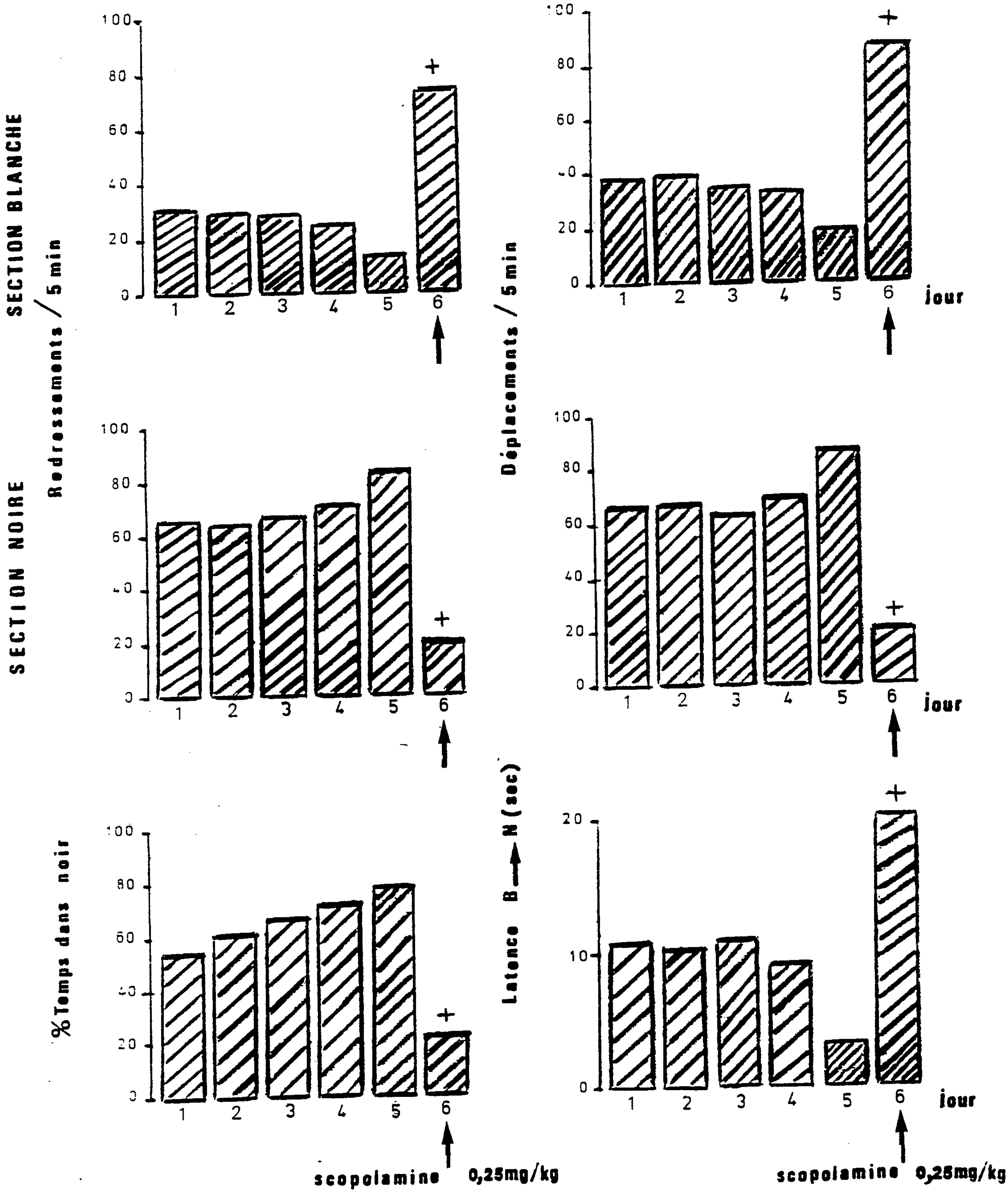


PROCESSUS D'HABITUATION DE SOURIS ADULTES JEUNES

EFFET DE LA SCOPOLAMINE

figure 1

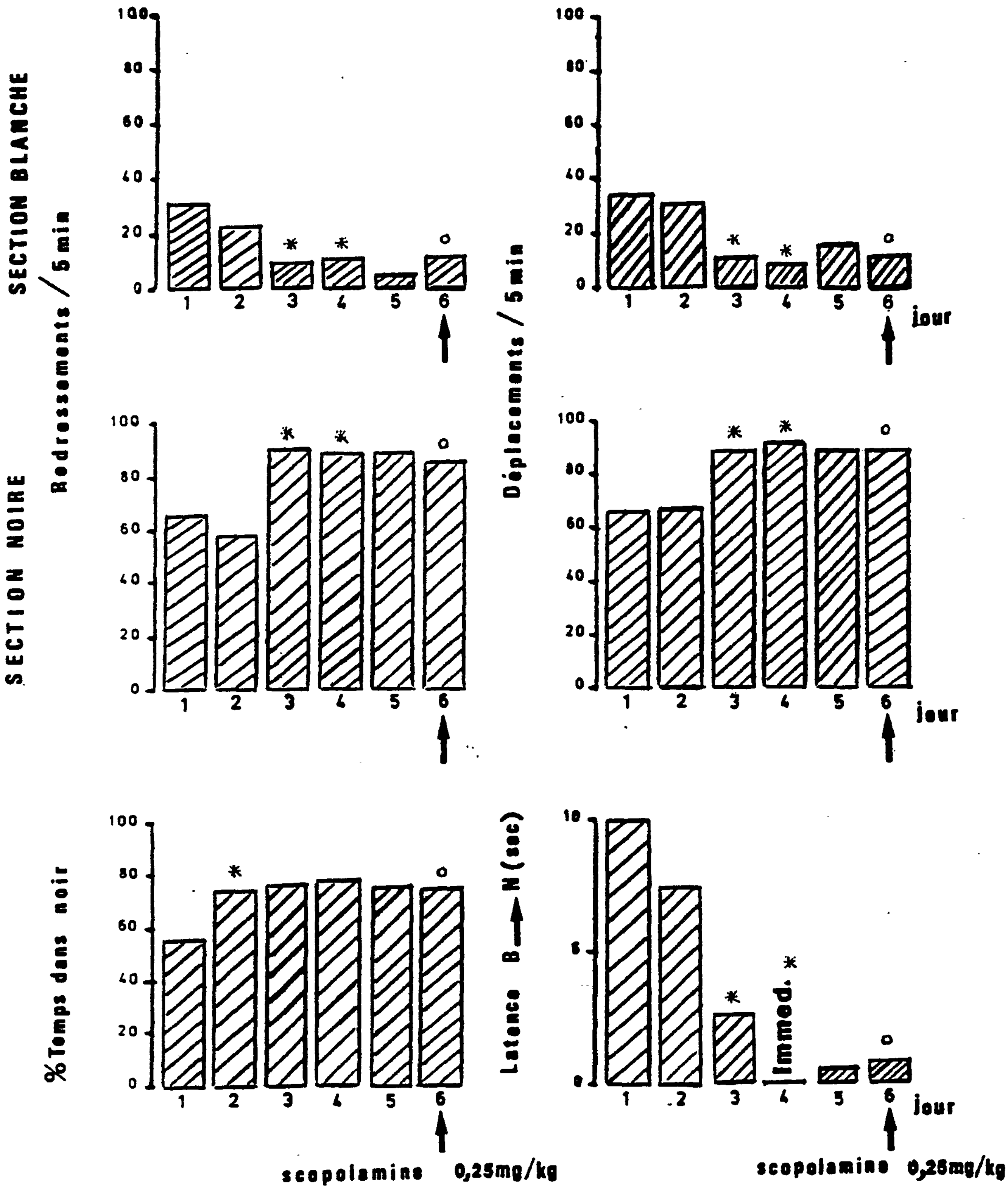


EFFET DU COMPOSE 1 SUR LE PROCESSUS D HABITUATION DE SOURIS ADULTES JEUNES ET SUR LES PERTURBATIONS CAUSEES PAR LA SCOPOLAMINE

figure 2

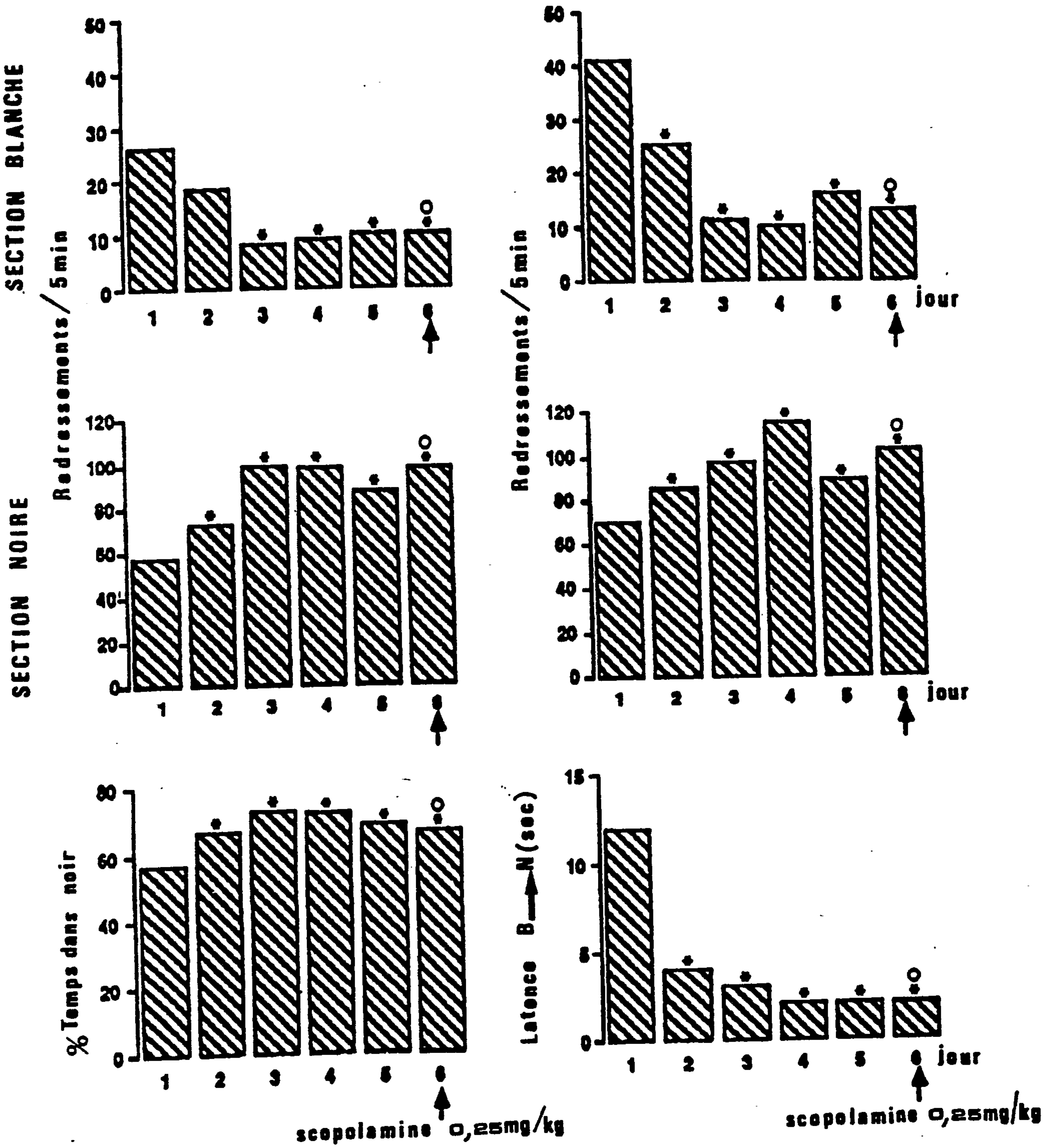


EFFET DU COMPOSE 2 SUR LE PROCESSUS D'HABITUATION DE SOURIS ADULTES JEUNES ET SUR LES PERTURBATIONS CAUSEES PAR LA SCOPOLAMINE

figure 3

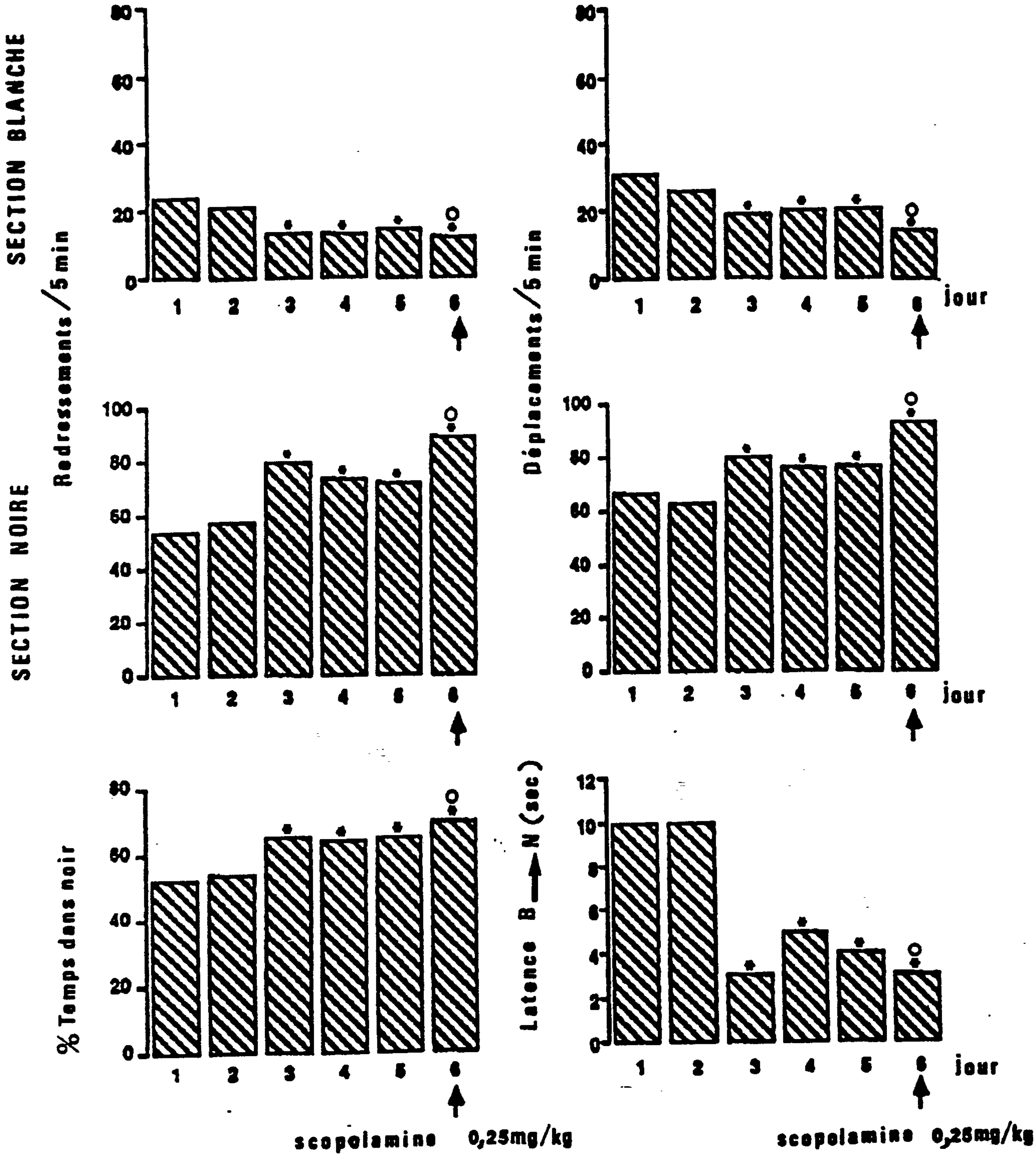


EFFET DU COMPOSE 3 SUR LE PROCESSUS D'HABITUATION DE SOURIS ADULTES JEUNES ET SUR LES PERTURBATIONS CAUSEES PAR LA SCOPOLAMINE

figure 4

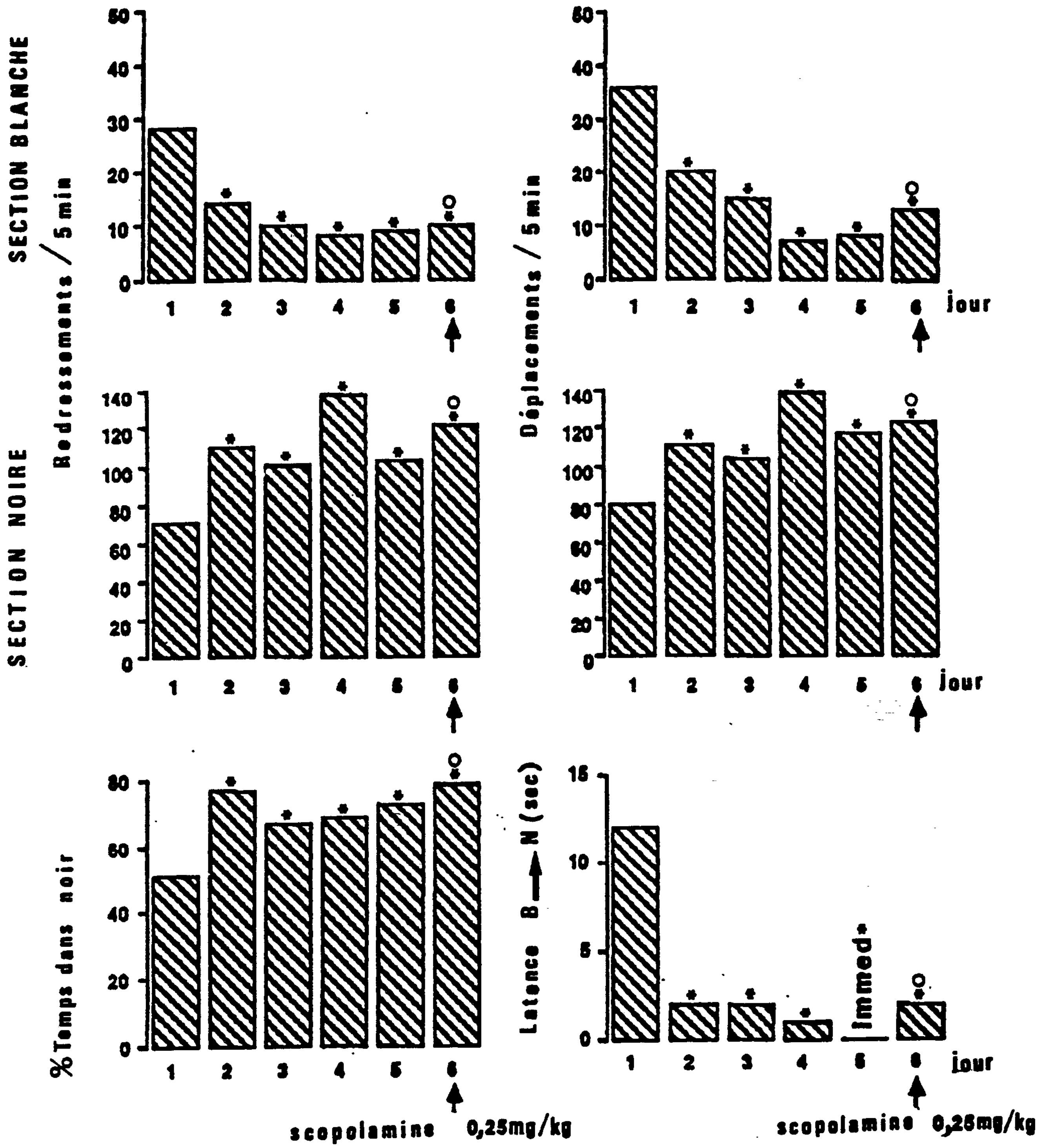


EFFET DU COMPOSE 4 SUR LE PROCESSUS D'HABITUATION DE SOURIS ADULTES JEUNES ET SUR LES PERTURBATIONS CAUSEES PAR LA SCOPOLAMINE

figure 5

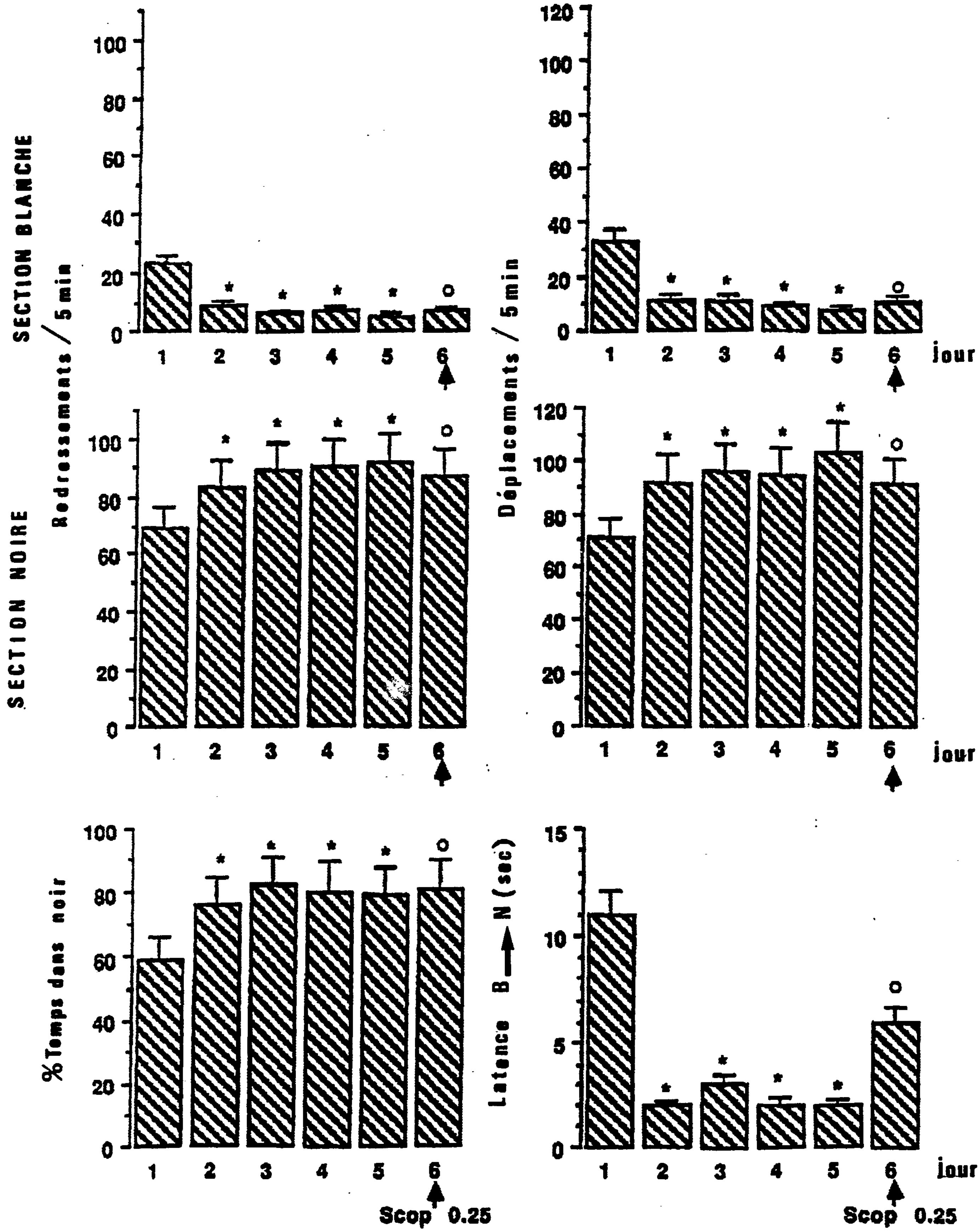


EFFET DU COMPOSE 5 SUR LE PROCESSUS D'HABITUATION DE SOURIS ADULTES JEUNES ET SUR LES PERTURBATIONS CAUSEES PAR LA SCOPOLAMINE

figure 6

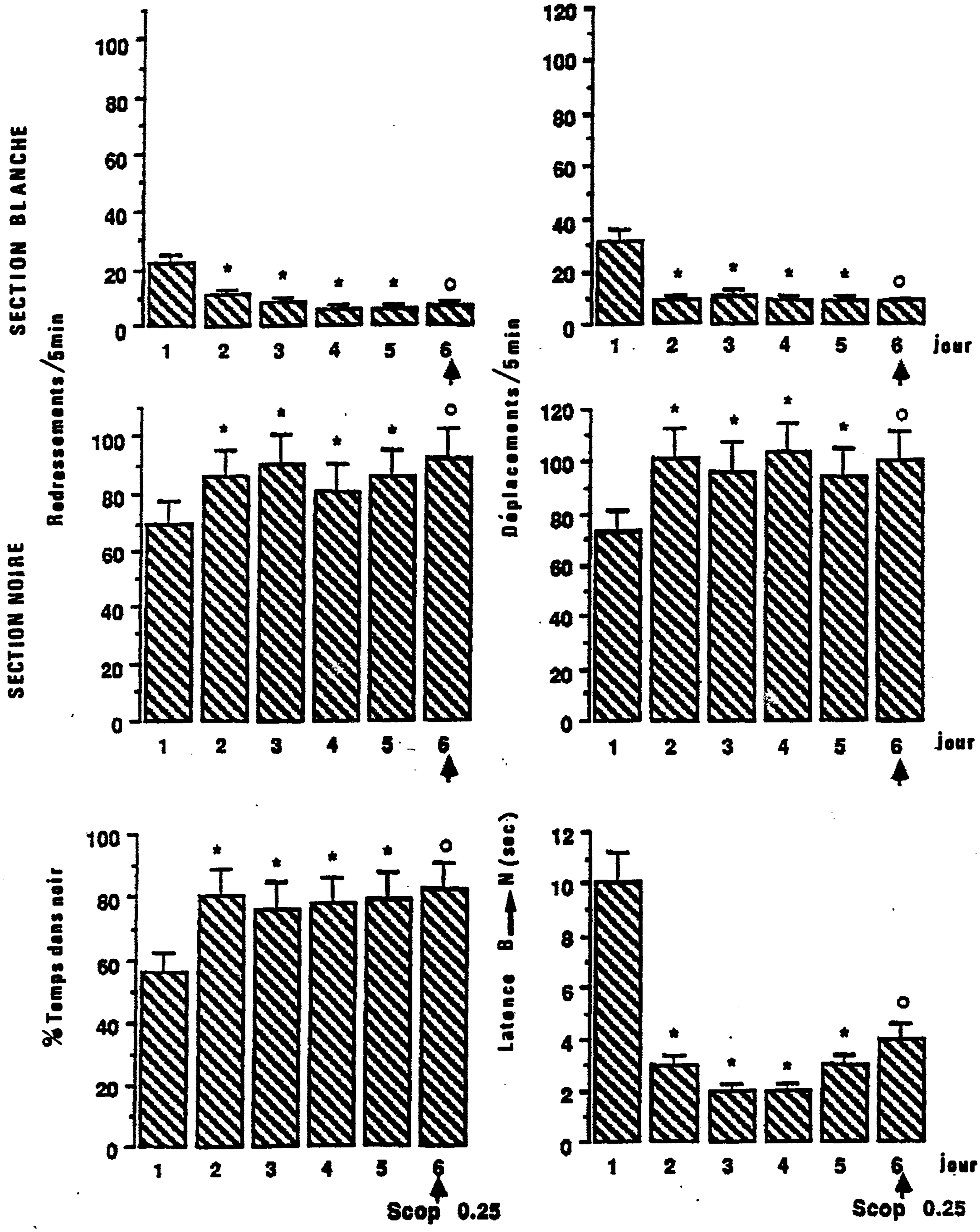


EFFET DU COMPOSE 6 SUR LE PROCESSUS D'HABITUATION DE SOURIS ADULTES JEUNES ET SUR LES PERTURBATIONS CAUSEES PAR LA SCOPOLAMINE

figure 7

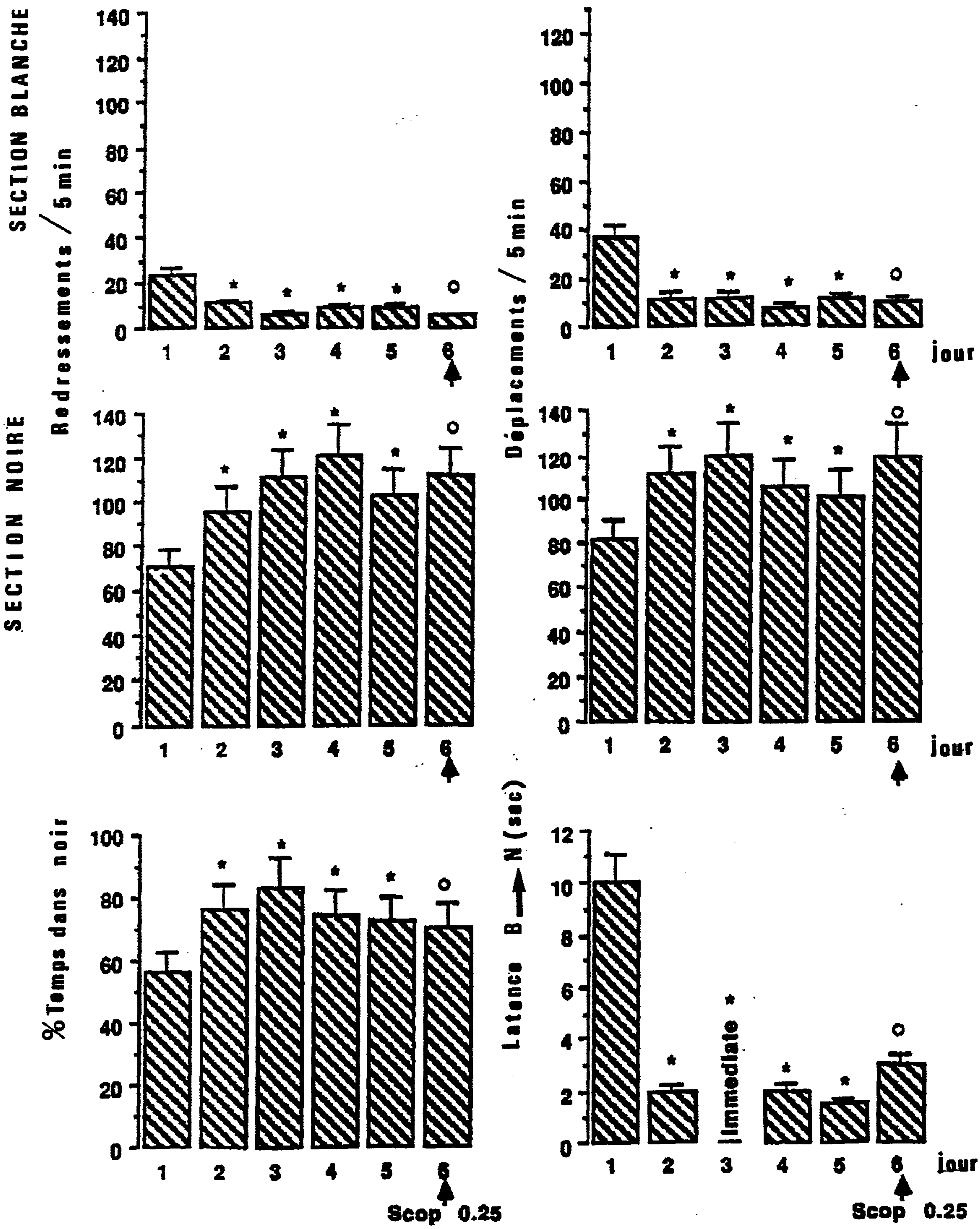


EFFET DU COMPOSE 7 SUR LE PROCESSUS D'HABITUATION DE SOURIS ADULTES JEUNES ET SUR LES PERTURBATIONS CAUSEES PAR LA SCOPOLAMINE

figure 8

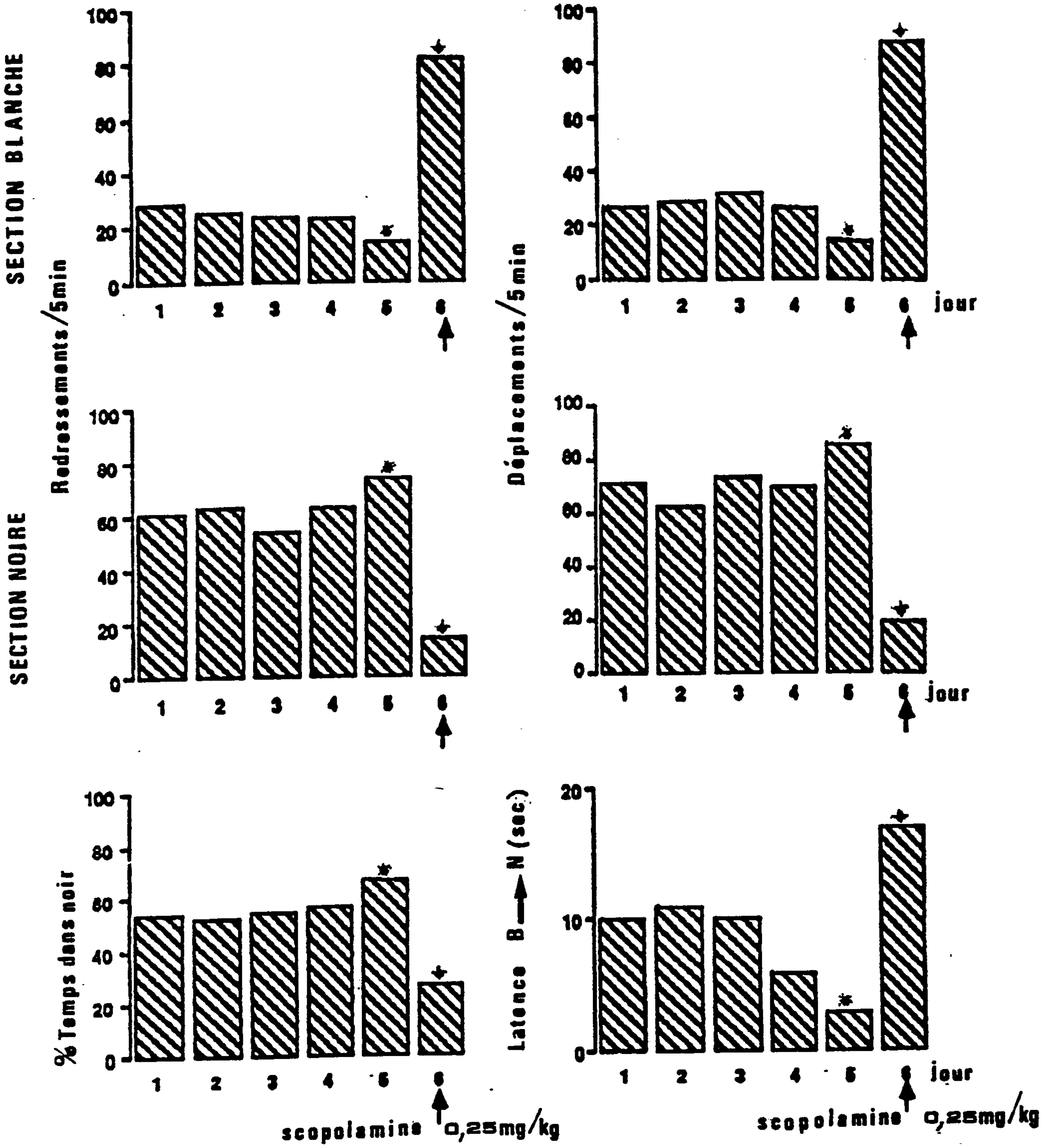


EFFET DU COMPOSE A (10mg/kg) SUR LE PROCESSUS D'HABITUATION DE SOURIS ADULTES JEUNES ET SUR LES PERTURBATIONS CAUSEES PAR LA SCOPOLAMINE

figure 9

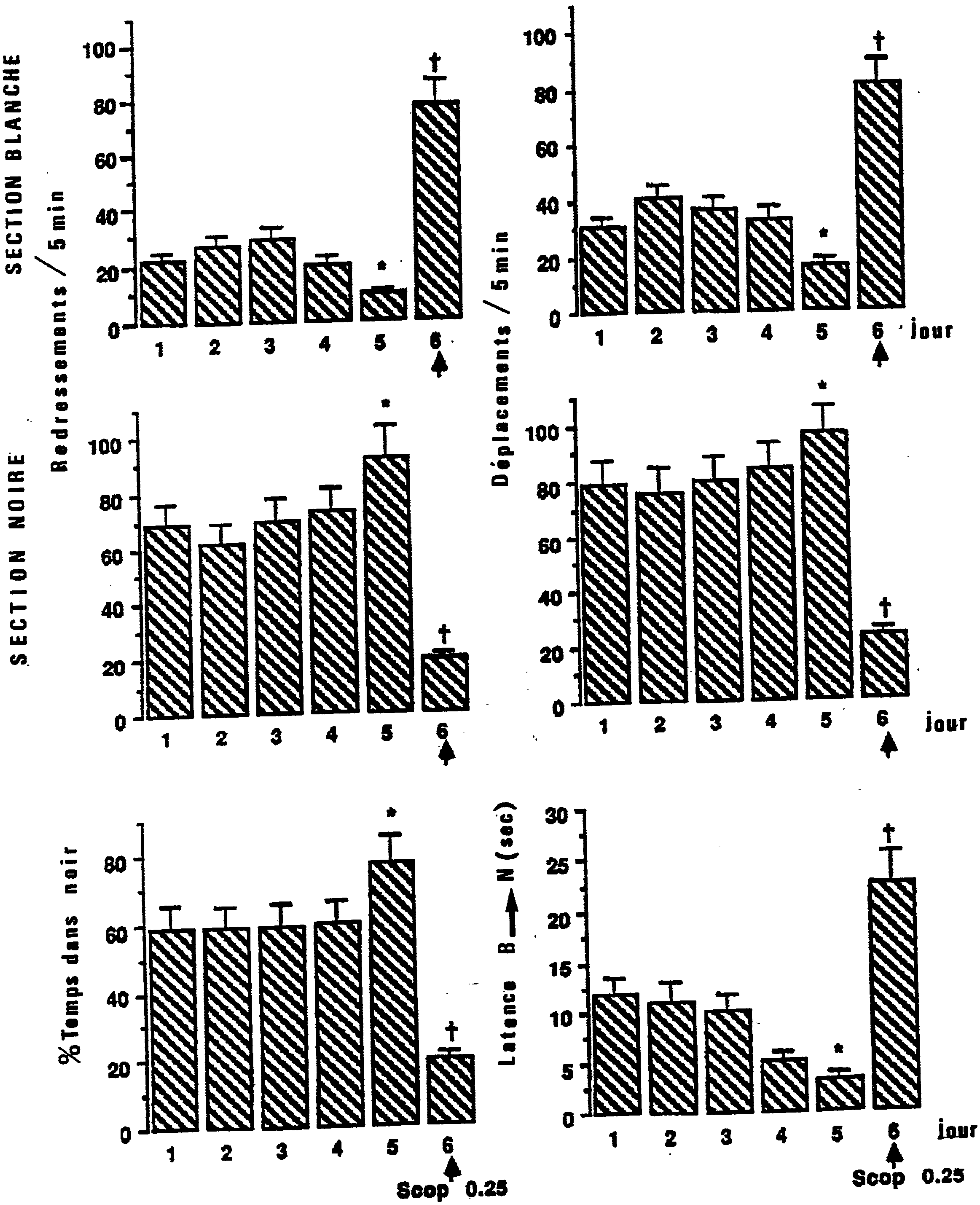


EFFET DU COMPOSE A (1µg/kg) SUR LE PROCESSUS D'HABITUATION DE SOURIS ADULTES JEUNES ET SUR LES PERTURBATIONS CAUSEES PAR LA SCOPOLAMINE

figure 10

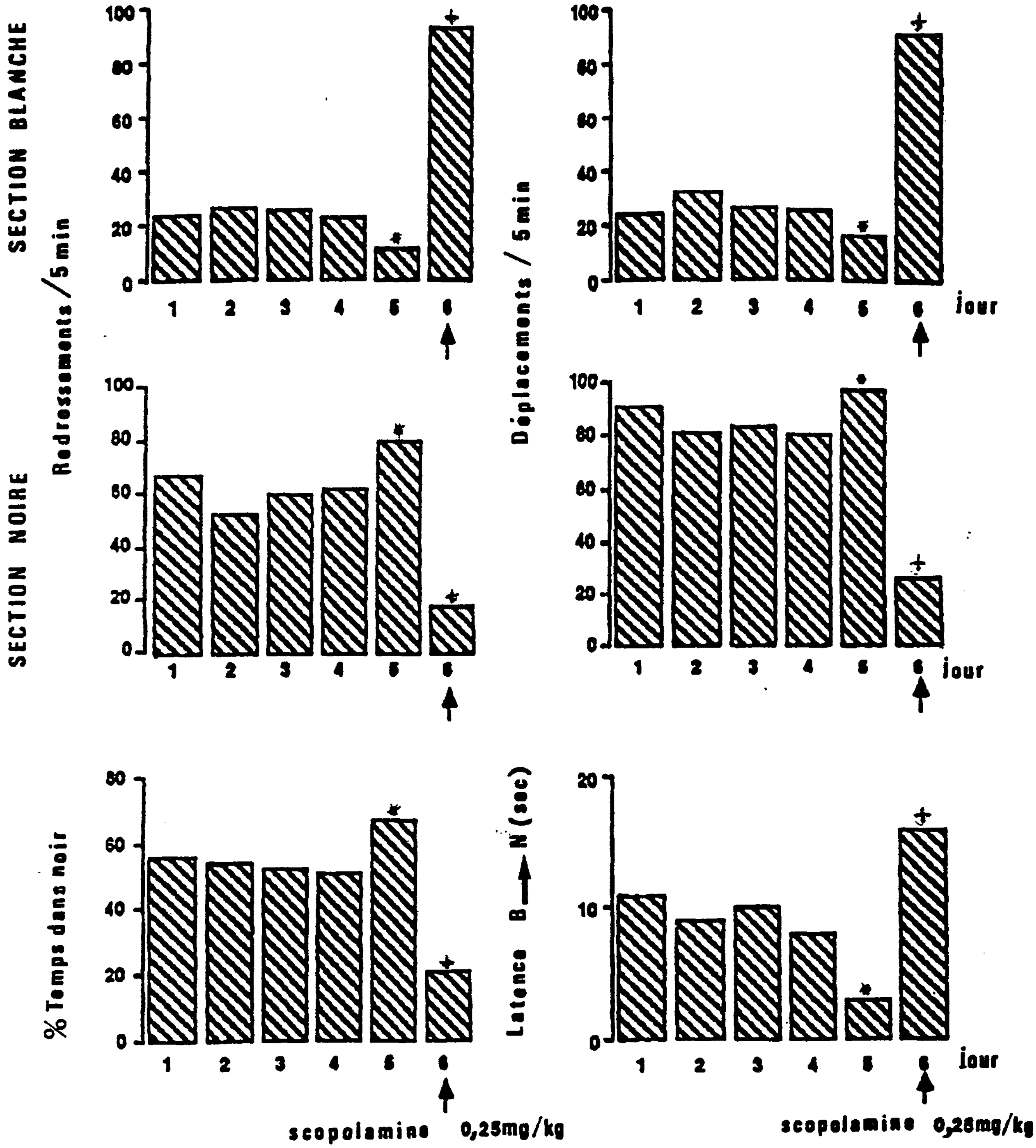


EFFET DU COMPOSE B (10mg/kg) SUR LE PROCESSUS D'HABITUATION DE SOURIS ADULTES JEUNES ET SUR LES PERTURBATIONS CAUSEES PAR LA SCOPOLAMINE

figure 11

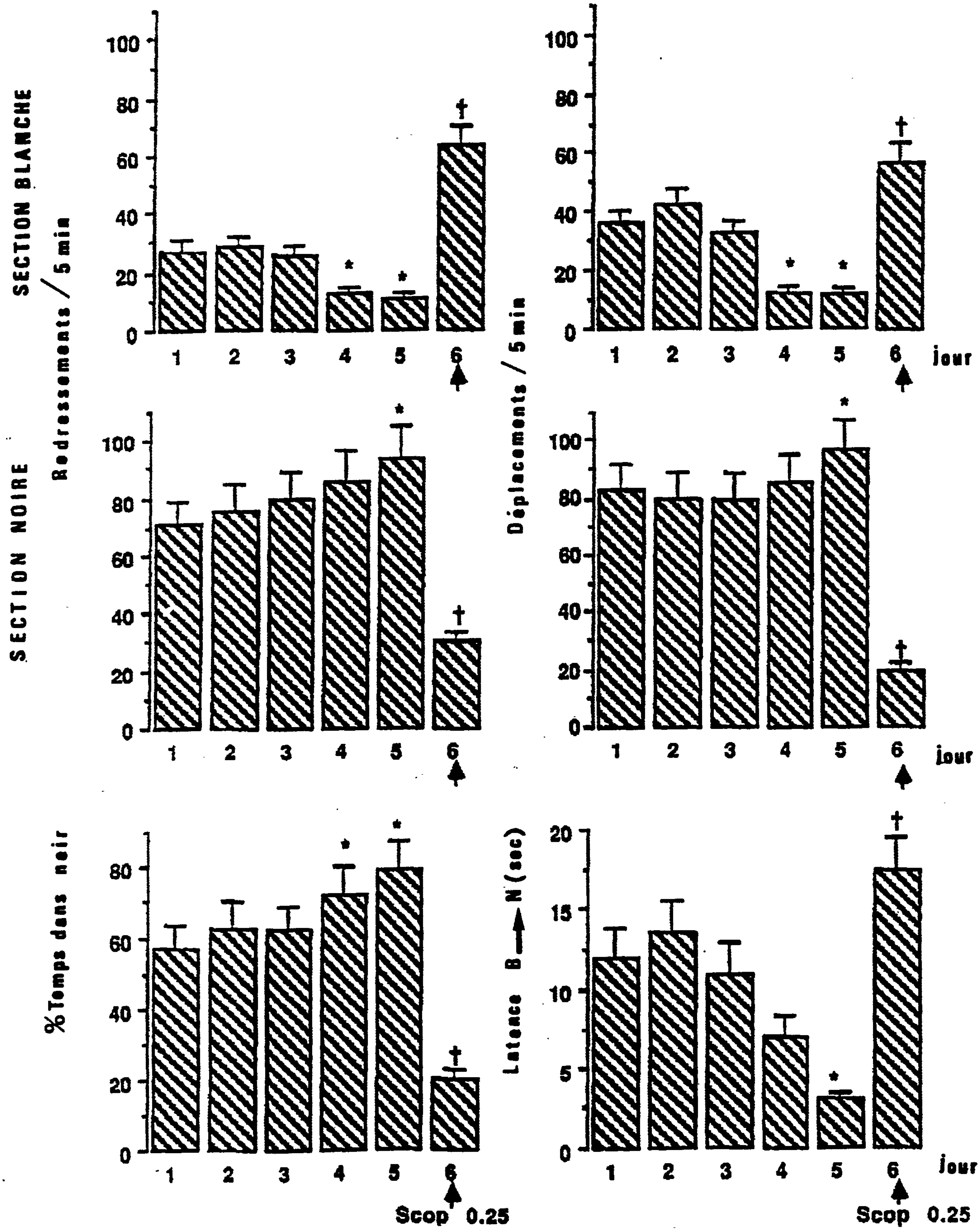


EFFET DU COMPOSE B (1 µg/kg) SUR LE PROCESSUS D'HABITUATION DE SOURIS ADULTES JEUNES ET SUR LES PERTURBATIONS CAUSEES PAR LA SCOPOLAMINE

figure 12

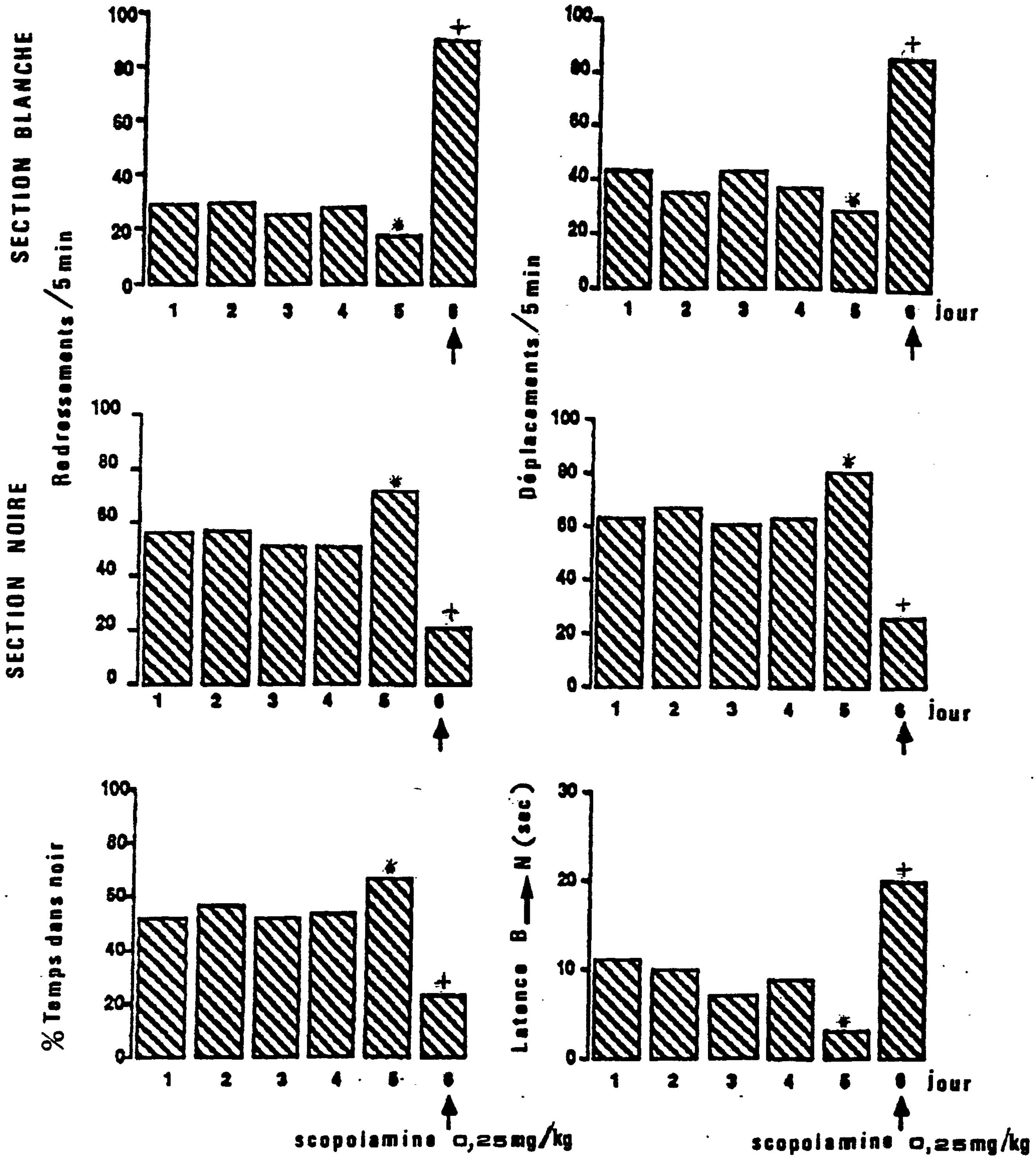


EFFET DU COMPOSE C SUR LE PROCESSUS D'HABITUATION DE SOURIS ADULTES JEUNES ET SUR LES PERTURBATIONS CAUSEES PAR LA SCOPOLAMINE

figure 13

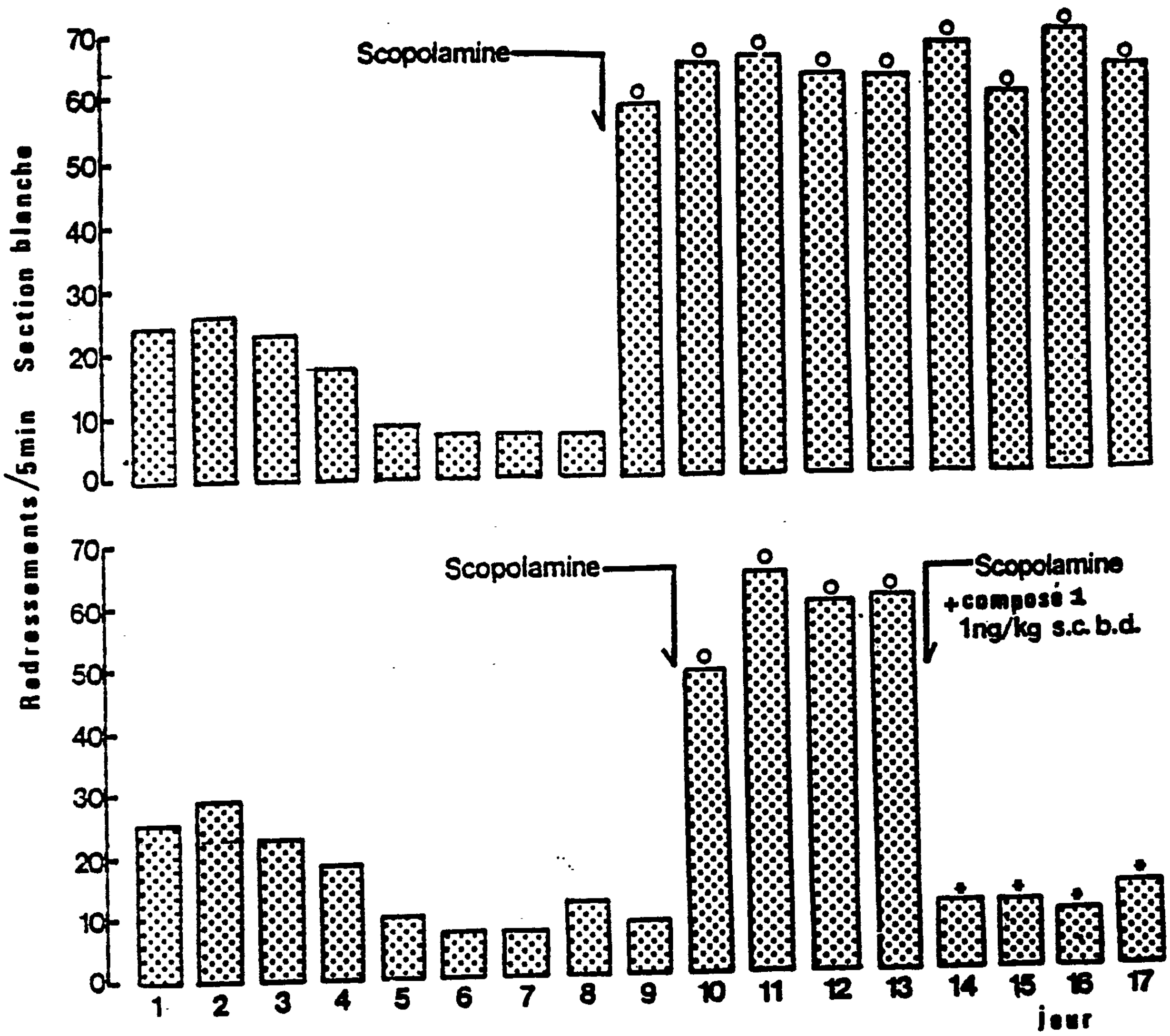


n = 5 E.S.M.< 12,3

°p < 0,001 ( Perturbation par la scopolamine )

PERTURBATIONS DE L'HABITUATION PAR LA SCOPOLAMINE CHEZ LA SOURIS

ANTAGONISME DES PERTURBATIONS PAR LE COMPOSE 1

figure 14

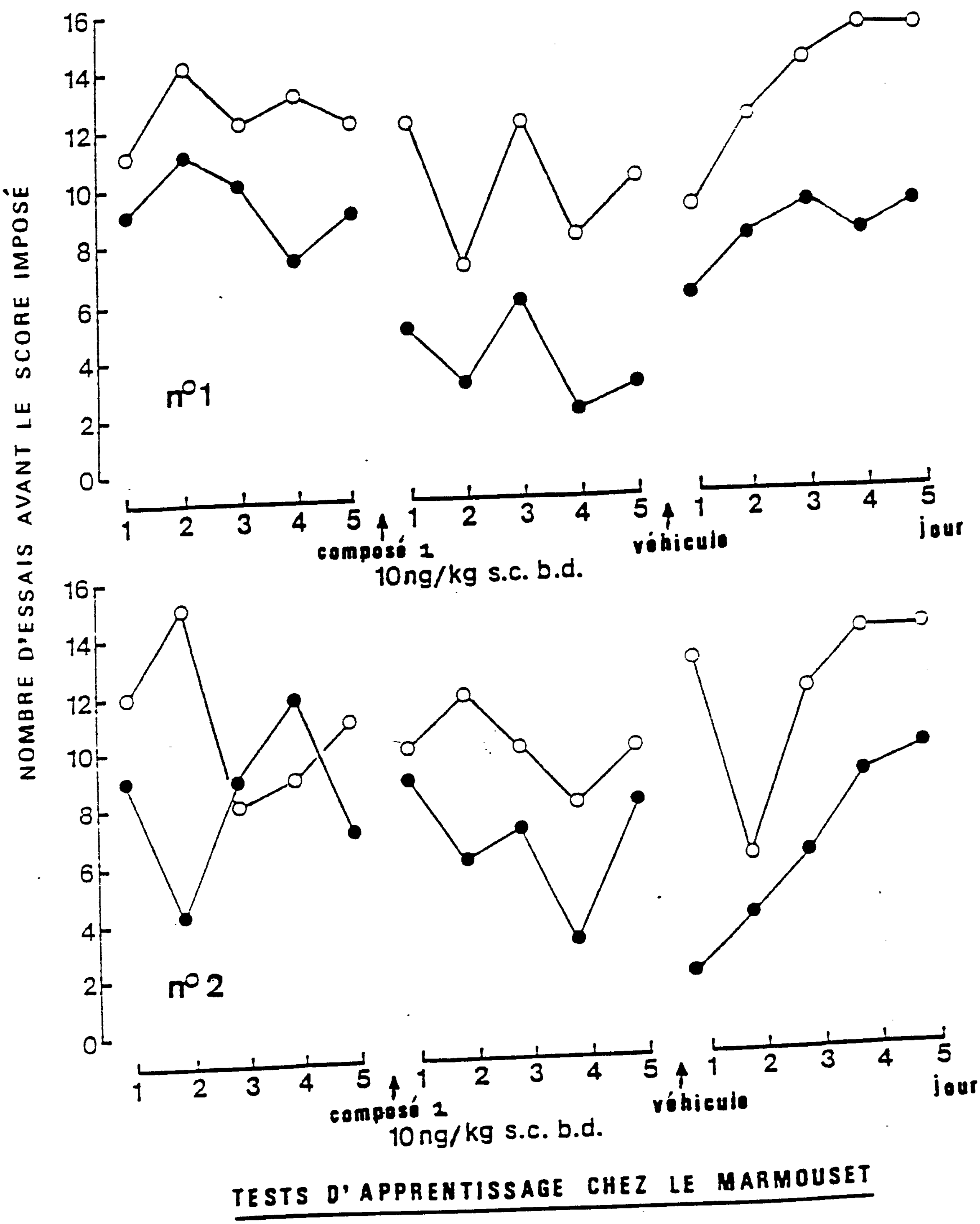
NOMBRE D'ESSAIS AVANT LE SCORE IMPOSÉ
16
14
12
10
8
6
4
2
0
n°1
1 2 3 4 5
composé 1
10ng/kg s.c. b.d.
1 2 3 4 5
véhicule
1 2 3 4 5
jour
n°2
1 2 3 4 5
composé 1
10ng/kg s.c. b.d.
1 2 3 4 5
véhicule
1 2 3 4 5
jour
TESTS D'APPRENTISSAGE CHEZ LE MARMOUSET

figure 15

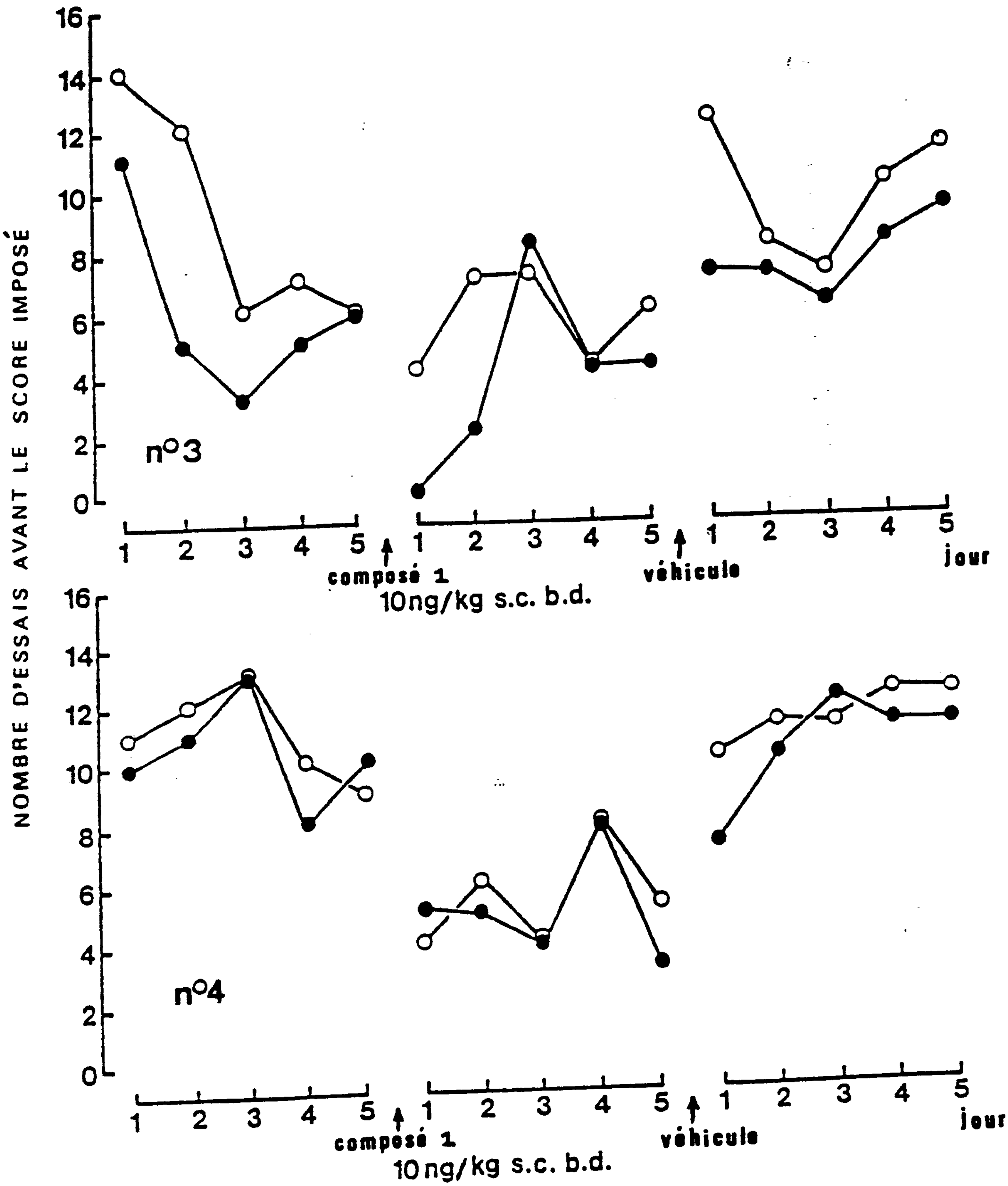


TESTS D'APPRENTISSAGE CHEZ LE MARMOUSET

figure 16